# EUROPEAN PATENT SPECIFICATION

(11) **EP 1 743 174 B1**
(45) Date of publication and mention of the grant of the patent: **20.03.2013**
(21) Application number: 05761586.6
(22) Date of filing: 19.04.2005
(51) Int. Cl.: G01N 33/58, A61K 49/00, G01N 33/543, B82Y 15/00

(54) **MULTIMODALITY NANOSTRUCTURES, METHODS OF FABRICATION THEREOF, AND METHODS OF USE THEREOF**
MULTIMODALE NANOSTRUKTUREN, VERFAHREN ZU DEREN HERSTELLUNG UND VERWENDUNG
NANOSTRUCTURES DE MULTIMODALITE, PROCEDES DE FABRICATION ET D'UTILISATION ASSOCIES

(30) Priority: 20.04.2004 US 563865 P; 19.04.2005 US 109368
(43) Date of publication of application: 17.01.2007
(60) Divisional application: 10192116.1
(73) Proprietor: Emory University, Atlanta, Georgia 30322 (US)
(72) Inventor: NIE, Shuming, Atlanta, GA 30319 (US); GAO, Xiaohu, Shoreline, WA 98155 (US)
(74) Representative: Dey, Michael
(86) International application number: PCT/US2005/013264
(87) International publication number: WO 2005/102396

(56) References cited:
- WO-A-03/003015
- WO-A2-03/064620
- US-B1- 6 548 264
- KIRCHER M F ET AL: "A MULTIMODAL NANOPARTICLE FOR PREOPERATIVE MAGNETIC RESONANCE IMAGING AND INTRAOPERATIVE OPTICAL BRAIN TUMOR DELINEATION" CANCER RESEARCH, AMERICAN ASSOCIATION FOR CANCER RESEARCH, BALTIMORE, MD, vol. 63, 1 December 2003 (2003-12-01), pages 8122-8125, XP008061528 ISSN: 0008-5472

## Description

### BACKGROUND

Recent advances in bioanalytical sciences and bioengineering have led to the development of DNA chips, miniaturized biosensors, and microfluidic devices. In addition, applications benefiting from fluorescent labeling include medical (and non-medical) fluorescence microscopy, histology, flow cytometry, fundamental cellular and molecular biology protocols, fluorescence in situ hybridization, DNA sequencing, immunoassays, binding assays, and separation. These enabling technologies have substantially impacted many areas in biomedical research, such as gene expression profiling, drug discovery, and clinical diagnostics.

Imaging techniques such as fluorescence reflectance imaging, fluorescence mediated tomography, bioluminescence imaging, intravital microscopy, X-ray computed tomography (CT), magnetic resonance imaging (MRI), ultrasound (ULT), single photon emission computed tomography (SPECT) positron emission tomography (PET), and the like, can be used to study various biological systems and events. However, each imaging technique has strengths and weaknesses (*e.g*., cost, sensitivity, resolution, safety, imaging depth, and the like). Therefore, there is a need in the industry for more accurate, sensitive, and broader method of detection that combines the strengths of the imaging techniques.

WO 03/064620 relates to a substrate design for targeting and/or inhibiting hepsin enzyme activity. Hepsin substrates, prodrugs, diagnostics and inhibitors as well as methods involving the hepsin substrate and vectors useful in expressing hepsin are disclosed. These substrates, inter alia, can be used for the treatment of cancers or diagnostic approaches. The substrate is a hepsin cleavable molecule having a hepsin cleavage site. Attached to this hepsin cleavable molecule are different moieties for different purposes of the invention, e.g. label moieties, inhibitors or cytotoxic moieties. The label moieties include fluorescent or luminescent moities. This label can be attached to the hepsin cleavable molecule either directly or via a linker or spacer molecule.

Kircher et al. (Cancer Research, 2003) describe the use of multimodal nanoparticles with two detectable functionalities (Cy5.5 conjugated to an iron oxide core) as a preoperative MRI contrast agent and intraoperative optical probe for the determination of brain tumor margins.

Tan et al. (US 6,548,264 B1) discloses silica-coated nanoparticles and a process for producing them. Those particles can be used in a variety of applications like as dye-doped particles or metal particles.

### SUMMARY

Briefly described, embodiments of this disclosure include multimodality nanostructures, methods of forming the multimodality nanostructures, and methods of using the multimodality nanostructures. One exemplary multimodality nanostructure, among others, includes at least one nanospecies having a first detectable functionality and a second molecule having a second detectable functionality, wherein the nanospecies is a quantum dot, and the second molecule is a contrasting agent and not a quantum dot that is attached to the nanospecies, wherein the contrasting agent is selected from the following: a magnetic compound, paramagnetic compound, radioisotope, fluorescent compound, and an organic dye, wherein the first detectable functionality and the second detectable functionality are different, and wherein the first detectable functionality is an optical functionality and the second detectable functionality is selected from the following: an optical functionality, a magnetic functionality, an x-ray functionality, and a radioactive functionality.

One exemplary method of preparing a multimodality nanostructure as described above, among others includes: providing a nanospecies having a first detectable functionality; and disposing a second molecule to the nanospecies, wherein the nanospecies is a quantum dot and the second molecule has a second detectable functionality, and wherein the first detectable functionality and the second detectable functionality are different.

One exemplary use of a nanostructure as described above is for the manufacture of a diagnostic agent for the detection of the target in a subject, wherein the target is selected from the following: polypeptides, nucleic acids, polysaccharides, sugars, fatty acids, steroids, purines, pyrimidines, drugs, ligands, or combinations thereof.

### BRIEF DESCRIPTION OF THE DRAWINGS

Many aspects of the disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.
FIG. 1 illustrates an embodiment of a multimodality nanostructure.
FIG. 2 illustrates an embodiment of a multimodality nanostructure.
FIG. 3 illustrates an embodiment of a multimodality nanostructure.
FIG. 4 illustrates an embodiment of a multimodality nanostructure.
FIG. 5 illustrates an embodiment of a multimodality nanostructure.
FIG. 6 illustrates an embodiment of a multimodality nanostructure.
FIG. 7 illustrates an embodiment of a multimodality nanostructure.
FIG. 8 illustrates an embodiment of a multimodality nanostructure.
FIG. 9 illustrates an embodiment of a multimodality nanostructure.
FIG. 10 illustrates an embodiment of a multimodality nanostructure.
FIG. 11 illustrates an embodiment of a multimodality nanostructure.
FIG. 12 illustrates an embodiment of a multimodality nanostructure.
FIG. 13 illustrates an embodiment of a multimodality nanostructure.
FIG. 14 illustrates an embodiment of a multimodality nanostructure.
FIG. 15 illustrates an embodiment of a multimodality nanostructure.
FIG. 16 illustrates that the polyamines enhance the fluorescence intensity of the multimodality nanostructure.
FIG. 17 is a graph that illustrates the degree of modification of amines on an embodiment of the multimodality nanostructure shown in FIG. 16.
FIG. 18 illustrates MRI images before and after injection of the multimodality nanostructure shown in FIG. 16 into a mouse and a fluorescence image after injection.

### DETAILED DESCRIPTION

In accordance with the purpose(s) of the present disclosure, as embodied and broadly described herein, embodiments of the present disclosure, in one aspect, relate to multimodality nanostructures (hereinafter nanostructures), methods of fabricating the nanostructures, and methods of using the nanostructures. The nanostructures are distinguishable because they include components having at least two different detectable functionalities, and therefore, can be individually detected using two or more imaging techniques. The imaging techniques can include, but is not limited to, optical systems, magnetic systems, x-ray systems, nuclear systems, positron emission tomography (PET) imaging systems, ultrasound systems, and the like. In particular, the imaging techniques can include, but are not limited to, fluorescence mediated tomography, bioluminescence imaging, intravital microscopy, X-ray computed tomography (CT), magnetic resonance imaging (MRI), ultrasound (ULT), single photon emission computed tomography (SPECT), PET, and combinations thereof.

The nanostructures can be used in many areas such as, but not limited to, multimodality imaging contrast agents (*in vitro* and *in vivo*), biosensing, biolabeling, gene expression studies, protein studies, medical diagnostics, diagnostic libraries, microfluidic systems, delivery vehicles, nano-tweezers (*e.g*., the magnetic property can be used for nano-tweezers movement, while the optical property can be used for imaging, detection, and/or visualization) self-assembly, and lithography and patterning.

In addition, the nanostructures can be modified (*e.g*., attach biomolecule probes to the nanostructure) so that the nanostructures interact with certain target molecules, which allow detection of the target molecules using one or more imaging techniques. For example, the nanostructures in combination with spectral imaging can be used for multiplexed, optical imaging and detection of genes, proteins, and small-molecule drugs in single living cells, while MRI, PET, and the like, allow imaging of magnetic and radioactive compounds. Since each imaging technique has strengths and weaknesses, using multimodality nanostructures can be used to combine two or more of the strengths of the imaging techniques.

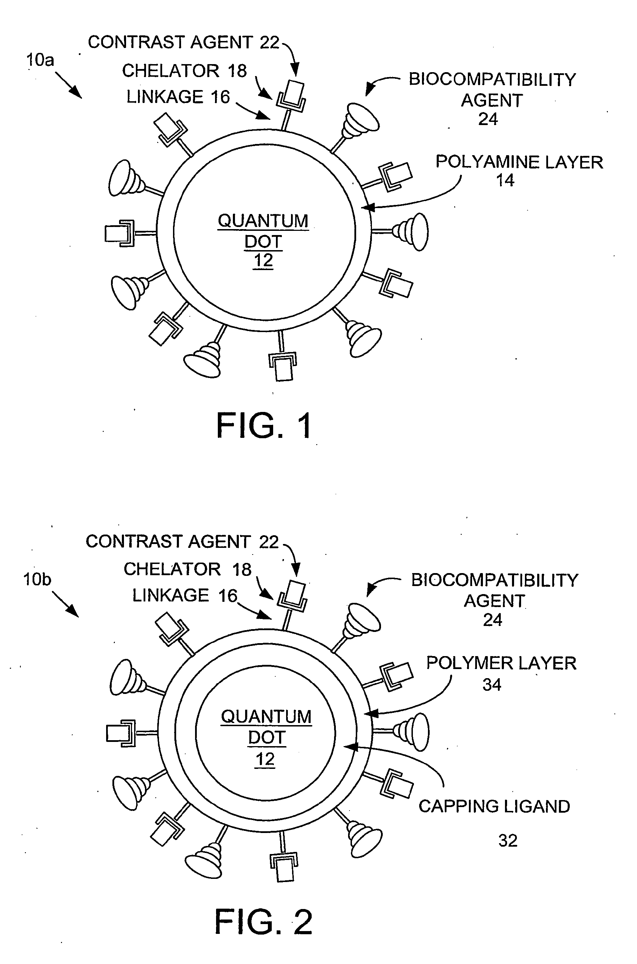

Embodiments of the nanostructure include, but are not limited to, at least one nanospecies having a first detectable functionality; and a second molecule having a second detectable functionality, wherein the nanospecies is a quantum dot, and the second molecule is a contrasting agent and not a quantum dot that is attached to the nanospecies, wherein the contrasting agent is selected from the following: a magnetic compound, paramagnetic compound, radioisotope, fluorescent compound, and an organic dye, wherein the first detectable functionality and the second detectable functionality are different, and wherein the first detectable functionality is an optical functionality and the second detectable functionality is selected from the following: an optical functionality, a magnetic functionality, an x-ray functionality, and a radioactive functionality. A few illustrative embodiments are disclosed in FIGS. 1 through 15 and in Examples 1-11 below.

The detectable functionality of the nanospecies is an optical functionality and the detectable functionality of the contrasting agent can include, but is not limited to, optical functionality, a magnetic functionality, an x-ray functionality, and a radioactive functionality. As mentioned above, the detectable functionality can be detected using two or more imaging techniques. The imaging technique can include, but is not limited to, optical imaging systems (*e.g*., fluorescence, luminescence, and scattering), magnetic imaging systems, x-ray imaging systems, nuclear imaging systems, positron emission tomography (PET) imaging systems, ultrasound imaging systems, and combinations of such imaging systems. The functionality can be measured simultaneously or sequentially using one or more of the imaging systems.

The nanospecies includes semiconductor nanoparticles (semiconductor quantum dots). Metal and metal oxide nanoparticles (e.g., iron, gold, silver, copper, titanium, nickel, platinum, palladium, oxides thereof (*e.g*., Cr₂O₃, Co₃O₄, NiO, MnO, Fe₂O₃, Fe₃O₄, FePt, FeCo, FeAl, FeCoAl, CoFe₂O₄, and MnFeO₄), alloys thereof, oxynitrides of each, clusters of each, cluster composites of each, and combinations thereof), metalloid and metalloid oxide nanoparticles, the lanthanide series metal nanoparticles, and combinations thereof are also described. In an embodiment, the nanospecies is a single structure, while in other embodiments, the nanospecies includes two or more structures (*e.g*., clusters, agglomerates, and the like).

The diameter of the nanostructure including contrast agents and other components can vary significantly depending on the particular application. In general, the diameter of the nanostructure is about 1 to 100 nm, about 2 to 50 nm, and about 2 to 30 nm.

In general, the size of the nanospecies depends on the nanospecies, the presence of a coating(s) on the nanospecies, the size and number of the contrast agents as well as the as the presence of other components such as bio-compatibility compounds and probes. Typically, when the nanospecies is a quantum dot, the diameter of the quantum dot is about 1 nanometer (nm) to 30 nm, about 1 to 25 nm, about 1 to 20 nm, about 1 to 15 nm, and about 1 to 10 nm.

The diameter of the clusters and/or agglomerates of the nanospecies varies depending on the number of nanospecies and the presence of compounds on the nanospecies.

In particular, semiconductor quantum dots are described in more detail below and in U.S. Patent 6,468,808 and International Patent Application WO 03/003015,

Furthermore, the magnetic nanoparticles (*e.g*., those having magnetic, paramagnetic, and superparamagnetic properties) can include, but are not limited to, cobalt, iron nanoparticles, iron composite nanoparticles, oxides of each, alloys, clusters of each, or combinations thereof. In particular, the magnetic nanoparticles can include superparamagnetic iron oxide nanoparticles (*e.g*., iron oxide nanoparticles such as Fe₃O₄, Fe₂O₃). Additional paramagnetic compounds can include, but are not limited to, Cr, Mn, Fe, Cu, Eu, Gd, Dy, oxides thereof, or combinations thereof.

In an embodiment, the nanospecies can be covered in part, substantially covered, or completely covered by one or more layers of a material, such as a polymer or other compound. In an embodiment, the nanospecies can have a hydrophobic protection structure that encapsulates the nanospecies, while in another embodiment, the nanospecies is covered in a polyamine layer (*e.g*., polylysine, polyarginine, chitosan, polyallylamine, polyethylenimine, etc.). In general, the polyamine layer includes a plurality of monomer units. The hydrophobic protection structure includes a capping ligand and/or an amphiphilic copolymer (*e.g*., amphiphilic block copolymers, amphiphilic random copolymers, amphiphilic alternating copolymers, amphiphilic periodic copolymers, or combinations thereof). The layer or layers can be about 0.5 to 10 nm thick. Additional details are provided below.

The contrasting agent, a bio-compatibility compound, a probe, a linker, and/or a chelator compound can be attached directly to the nanospecies and/or attached to the layer disposed on the nanospecies. In addition, a probe (*e.g*., a polynucleotide, a polypeptide, a therapeutic agent, and/or a drug) can be attached to the nanospecies or the layer disposed on the nanospecies. The contrasting agent, the bio-compatibility compound, the probe, and/or the chelator compound, can be attached directly to the layer and/or via one or more linkers. The contrasting agent, the bio-compatibility compound, the probe, and/or the chelator compound can be attached in series via one or more linkers. Numerous embodiments of how the nanostructures can be configured is described below, but the following is only an illustrative list and other configurations are intended to be covered by this disclosure.

In an embodiment, the bio-compatibility compound can be attached directly or indirectly with the nanospecies and/or the hydrophobic protection layer. In still another embodiment, the nanospecies can include the bio-compatibility compound and a probe. The bio-compatibility compound and/or the probe are substantially disposed (*e.g*., attached to the surface of the hydrophobic protection structure and/or attached within the hydrophobic protection structure) on the hydrophobic protection structure.

In another embodiment, the nanostructure can include two or more nanospecies and/or two of more types of nanospecies. In addition, the nanostructure can include a hydrophobic protection structure having two or more polymers (*e.g*., two or more block copolymers). Further, the nanostructure can include multiple nanospecies and/or multiple polymers (*e.g*., block copolymers). In addition, the nanostructure can include two or more different types of probes having different functions. Furthermore, the nanospecies and the polymers (*e.g*., block copolymers) can be assembled into micro and macro structures.

In still another embodiment, the nanospecies can have a ligand coating layer (original ligands). For different types of compound depositions, the original ligands can be kept, partially replaced, or completely replaced by other types of ligands. The original and/or new ligands can include small molecules (*e.g*., trioctyl phosphine oxide, hexadecylamine, hexadecene, mercaptoacetic acid, oleic acid, citric acid, and derivatives thereof) or polymers (*e.g*., polymers, copolymers, surfactants, lipid, or derivatives thereof), which can adsorb nanoparticles and also provide a linkage for the contrasting agent, the bio-compatibility compound, the probe, and the chelator compound. The ligands and the nanospecies can interact through interactions such as, but not limited to, covalent bonds, hydrophobic interactions, hydrophilic interactions, or pi-stacking, *etc*., depending on the surface of nanospecies and the molecular structure of ligands.

In another embodiment, the nanospecies can be overcoated with another polymer on top of their original ligands, through interactions such as, but not limited to, hydrophobic interactions, hydrophilic interactions, covalent bond etc, then the compound is adsorbed onto the polymer via chelating, hydrophobic interactions, hydrophilic interactions, pi-stacking, covalent bond, combinations thereof, and the like.

Exemplary polymers for the overcoat can include those described above. For example, the overcoat polymer can include, but is not limited to, amphiphilic polymers, detergent and/or a lipid structure including detergent derivatives and lipid derivatives. The amphiphilic polymer can include, but is not limited to hydrocarbons and DTPA modified poly(acrylic acid), poly(maleic acid), poly(maleic anhydride), and the like. The detergents can include, but are not limited to, AOT, brij family, 1gepal family, triton family, SDS, or derivatives of each. In particular, the detergents can include, dioctyl sulfosuccinate sodium salt; polyethylene glycol dodecyl ether, (octylphenoxy) polyethoxyethanol, octylphenyl-polyethylene glycol, t-octylphenoxypolyethoxyethanol, polyethylene glycol tert-octylphenyl ether, 4-(1,1,3,3-tetramethylbutyl)phenyl-polyethylene glycol, dodecyl sulfate sodium salt, or glycolic acid ethoxylate octyl ether. Further, the block copolymer can include lipids such as, but not limited to, lipid-PEG, natural lipids, synthetic lipids, sphingolipids, or derivatives of each.

In particular, the block copolymer can include an ABC triblock structure having a poly-butylacrylate segment, a poly-ethylacrylate segment, or a polymethacrylic acid segment, for example. The block copolymer can include a diblock and/or triblock copolymer having two or more different poly-aliphatic-acrylate segments. In addition, the block copolymer can include a diblock and/or triblock copolymer having two or more poly-alkyl-acrylate segments.

In addition, the block copolymer can be used with, or in some embodiments replaced with, a detergent and/or a lipid. For example, the detergents can include, but are not limited to, AOT, brij family, 1gepal family, triton family, SDS, or derivatives of each. In particular, the detergents can include, dioctyl sulfosuccinate sodium salt, polyethylene glycol dodecyl ether, octylphenoxy polyethoxyethanol, octylphenyl-polyethylene glycol, t-octylphenoxypolyethoxyethanol, polyethylene glycol tert-octylphenyl ether, 4-(1,1,3,3-tetramethylbutyl)phenyl-polyethylene glycol, dodecyl sulfate sodium salt, or glycolic acid ethoxylate octyl ether. Further, the block copolymer can include lipids such as, but not limited to, lipid-PEG, natural lipids, synthetic lipids, sphingolipids, and derivatives of each.

The thickness of each layer disposed on nanospecies can vary significantly depending on the particular application. In general, the thickness is about 0.5 to 20 nm, about 0.5 to 15 nm, about 0.5 to 10 nm, and about 0.5 to 5 nm.

As mentioned above, the contrast agents, bio-compatibility compound, chelator compound, linkers, and probes, can be linked to the nanospecies using any stable physical and/or chemical association to the nanospecies directly or indirectly by any suitable means. For example, the component can be linked to the nanospecies using a covalent link, a non-covalent link, an ionic link, and a chelated link, as well as being absorbed or adsorbed onto the nanospecies. In addition, the component can be linked to the nanospecies through hydrophobic interactions, hydrophilic interactions, charge-charge interactions, π-stacking interactions, combinations thereof, and like interactions.

The bio-compatibility compound can include compounds such as, but not limited to, polyethylene glycol (MW about 500 to 50,000 and 1000 to 10,000); polypropylene glycol500 (MW of about 50,000), dextran, and derivatives such as amino-dextran and carboxy-dextran, and polysaccharides. The bio-compatibility compound can be attached directly or indirectly with the nanospecies and/or a layer (*e.g*., a hydrophobic protection layer or a polyamine layer) disposed on the nanospecies. The amount bio-compatibility compound disposed on the nanospecies can range widely depending on the application.

The chelator compound can include, but is not limited to, natural chelators and synthetic chelators. The natural chelators include, but are not limited to, carbohydrates (*e.g*., polysaccharides), organic acids with more than one coordination group, lipids, steroids, amino acids, peptides, phosphates, nucleotides, tetrapyrrols, ferrioxamines, lonophores (*e.g*., gramicidin, monensin, and valinomycin), and phenolics. The synthetic chelator include, but are not limited to, ammonium citrate dibasic, ammonium oxalate monohydrate, ammonium tartrate dibasic, ammonium tartrate dibasic solution, pyromellitic acid, calcium citrate tribasic tetrahydrate, ethylene glycol-bis(2-aminoethylether)-N,N,N,N-tetra acetic acid, sodium glycocholate, ammonium citrate dibasic, calcium citrate tribasic tetrahydrate, magnesium citrate tribasic, potassium citrate, sodium citrate monobasic, lithium citrate tribasic, sodium citrate tribasic, citric acid, N,N-dimethyldecylamine-N-oxide, N,N-dimethyldodecylamine-N-oxide, ammonium citrate dibasic, ammonium tartrate dibasic, ethylenediaminetetraacetic acid diammonium salt, potassium D-tartrate monobasic, N,N-dimethyldecylamine-N-oxide, N,N-dimethyldodecylamine-N-oxide, ethylenediaminetetraacetic acid dipotassium salt dihydrate, sodium tartrate dibasic, ethylenediaminetetraacetic acid, ethylenediaminetetraacetic acid disodium salt dihydrate, ethylenediaminetetraacetic acid, ethylenediaminetetraacetic acid tetrasodium salt hydrate, ethylenediaminetetraacetic acid tripotassium salt, ethylene glycol-bis(2-aminoethylether)-N,N,N,N-tetra acetic acid, ethylene glycol-bis(2-aminoethylether)-N,N,N,N-tetra acetic acid, potassium oxalate, sodium oxalate, ethylene glycol-bis(2-aminoethylether)-N,N,N,N-tetra acetic acid, ethylenediaminetetraacetic acid, ethylenediaminetetraacetic acid diammonium salt, ethylenediaminetetraacetic acid dipotassium salt dihydrate, ethylenediaminetetraacetic acid disodium salt dihydrate, ethylenediaminetetraacetic acid disodium salt dihydrate, ethylenediaminetetraacetic acid, ethylenediaminetetraacetic acid tetrasodium salt hydrate, ethylenediaminetetraacetic acid tripotassium salt, ethylenediaminetetraacetic acid trisodium salt trihydrate, ethylenediaminetetraacetic acid dipotassium salt dihydrate, ethylene glycol-bis(2-aminoethylether)-N,N,N,N-tetra acetic, ethylene glycol-bis(2-aminoethylether)-N,N,N,N-tetra acetic acid, sodium glycocholate, ethylene glycol-bis(2-aminoethylether)-N,N,N,N-tetra acetic acid, 5-sulfosalicylic acid, N,N-dimethyldodecylamine-N-oxide, magnesium citrate tribasic, magnesium citrate tribasic nonahydrate, ammonium oxalate monohydrate, potassium tetraoxalate, potassium oxalate, sodium oxalate, potassium citrate, ethylenediaminetetraacetic acid dipotassium salt dihydrate, potassium D-tartrate monobasic, potassium peroxodisulfate, potassium citrate monobasic, potassium citrate tribasic, potassium oxalate monohydrate, potassium peroxodisulfate, potassium sodium tartrate, potassium sodium tartrate tetrahydrate, potassium D-tartrate monobasic, potassium tetraoxalate dihydrate, pyromellitic acid hydrate, potassium sodium tartrate, potassium sodium tartrate, ethylenediaminetetraacetic acid disodium salt dihydrate, sodium citrate monobasic, sodium bitartrate, sodium tartrate dibasic, sodium bitartrate monohydrate, sodium citrate monobasic, sodium citrate tribasic dihydrate, sodium citrate tribasic, sodium glycocholate hydrate, sodium oxalate, sodium tartrate dibasic dihydrate, sodium tartrate dibasic, 5-sulfosalicylic acid dihydrate, ammonium tartrate dibasic, sodium tartrate dibasic, potassium D-tartrate monobasic, sodium bitartrate, potassium sodium tartrate, L-(+)-tartaric acid, ethylenediaminetetraacetic acid tetrasodium salt hydrate, L-(+)-tartanic acid, calcium citrate tribasic tetrahydrate, sodium glycocholate, lithium citrate tribasic, magnesium citrate tribasic, ethylenediaminetetraacetic acid tripotassium salt, sodium citrate tribasic, and ethylenediaminetetraacetic acid trisodium salt trihydrate. In particular, the chelator compound can include, but is not limited to, EDTA (ethylenediaminetetraacetic acid), DTPA (diethylenetriaminepentaacetate), DOPA (dihydroxyphenylalanine), and derivatives of each.

The amount of chelator compound disposed on the nanospecies can range widely depending on the particular application, but should not substantially alter the signal of the nanospecies and/or the physical properties of the nanostructure. It should be noted that the larger the number of chelator compounds disposed on the nanspecies, the stronger the signal from the compound chelated by the chelator compound.

The linker can include a functional group (*e.g*., an amine group) on the layer disposed on the nanospecies and/or the linker can include a separate compound attached to the nanospecies or the layer at one end and a chelator compound, contrast agent, bio-compatibility compound, or another linker at the other end. The linker can include functional groups such as, but not limited to, amines, carboxylic acids, hydroxyls, thios, and combinations thereof. The linker can include compounds such as, but not limited to, DTPA, EDTA, DOPA, EGTA, NTA, and combinations thereof. In an embodiment, the linker and the chelator compound are the same, but in other embodiments they can be different. The percentage of linkers attached to the chelator compound, contrast agent, and/or anther linker can be about 0.1 to 100%.

As indicated above, the nanostructure does include quantum dots such as, but not limited to, luminescent semiconductor quantum dots. In general, quantum dots include a core and a cap, however, uncapped quantum dots can be used as well. The "core" is a nanometer-sized semiconductor. While any core of the IIA-VIA, IIIA-VA, or IVA-IVA, IVA-VIA semiconductors can be used in the context of the present disclosure, the core must be such that, upon combination with a cap, a luminescent quantum dot results. A IIA-VIA semiconductor is a compound that contains at least one element from Group ILA and at least one element from Group VIA of the periodic table, and so on. The core can include two or more elements. In one embodiment, the core is a IIA-VIA, IIIA-VA, or IVA-IVA semiconductor that ranges in size from about 1 nm to about 40 nm, about 1 nm to 30, about 1 nm to 20 nm, about 1 nm to 10 nm. In another embodiment, the core is more preferably a IIA-VIA semiconductor and ranges in size from about 2 nm to about 10 nm. For example, the core can be CdS, CdSe, CdTe, ZnSe, ZnS, PbS, PbSe, or an alloy.

The "cap" is a semiconductor that differs from the semiconductor of the core and binds to the core, thereby forming a surface layer on the core. The cap can be such that, upon combination with a given semiconductor core a luminescent quantum dot results. The cap should passivate the core by having a higher band gap than the core. In one embodiment, the cap is a IIA-VIA semiconductor of high band gap. For example, the cap can be ZnS or CdS. Combinations of the core and cap can include, but.are not limited to, the cap is ZnS when the core is CdSe or CdS, and the cap is CdS when the core is CdSe. Other exemplary quantum does include, but are not limited to, CdS, ZnSe, CdSe, CdTe, CdSeₓTe₁₋ₓ, InAs, InP, PbTe, PbSe, PbS, HgS, HgSe, HgTe, CdHgTe, and GaAs. The cap is about 0.1 to 10 nm, about 0.1 to 5 nm, and about 0.1 to 2 nm.

The wavelength emitted (*i.e*., color) by the quantum dots can be selected according to the physical properties of the quantum dots, such as the size and the material of the nanocrystal. Quantum dots are known to emit light from about 300 nanometers (nm) to 2000 nm (*e.g*., UV, near IR, and IR). The colors of the quantum dots include, but are not limited to, red, blue, green, and combinations thereof. The color or the fluorescence emission wavelength can be tuned continuously. The wavelength band of light emitted by the quantum dot is determined by either the size of the core or the size of the core and cap, depending on the materials which make up the core and cap. The emission wavelength band can be tuned by varying the composition and the size of the QD and/or adding one or more caps around the core in the form of concentric shells.

The synthesis of quantum dots is well known and is described in U.S. Patent Nos. 5,906,670; 5,888,885; 5,229,320; 5,482,890; 6,468,808; 6,306,736; 6,225,198, etc., International Patent Application WO 03/003015,
and in many research articles. The wavelengths emitted by quantum dots and other physical and chemical characteristics have been described in US Patent 6,468,808 and International Patent Application WO 03/003015 and will not be described in any further detail. In addition, methods of preparation of quantum dots are described in US Patent 6,468,808 and International Patent Application WO 03/003015 and will not be described any further detail.

In an embodiment, the quantum dot can be substantially coated or coated with a polymer. The polymer can include, but is not limited to, a polyamine, a capping ligand, a hydrophobic protection layer, and combinations thereof. In particular, the polyamine can include, but is not limited to, polylysine. The coverage of the quantum dot with the polymer can range from about 1 to 100%. The thickness of the polymer layer can range from about 0.5 to 50 nm.

In another embodiment, the quantum dot can be capped with a capping ligand, which forms a layer on the quantum dot, and have a polymer layer disposed on the capping ligand. The capping ligand can include compounds such as, but not limited to, an O=PR₃ compound, an O=PHR2 compound, an O=PHR₁ compound, a H₂NR compound, a HNR₂ compound, a NR₃ compound, a HSR compound, a SR₂ compound, and combinations thereof. "R" can be a C₁ to C₁₈ hydrocarbon such as, but not limited to, linear hydrocarbons, branched hydrocarbons, cyclic hydrocarbons, substituted hydrocarbons (*e.g*., halogenated), saturated hydrocarbons, unsaturated hydrocarbons, and combinations thereof. Preferably, the hydrocarbon is a saturated linear C₄ to C₁₈ hydrocarbon, a saturated linear C₆ to C₁₈ hydrocarbon, and a saturated linear C₁₈ hydrocarbon. A combination ofR groups can be attached to P, N, or S. In particular, the capping ligand can be selected from, but is not limited to, tri-octylphosphine oxide, stearic acid, and octyldecyl amine, oleic acid, and derivatives thereof.

The polymer can include di- and/or tri-block copolymers. In addition, the polymer can be amphilphilic. Further, the polymer can include hydrocarbon side chains such as, but not limited to, 1-18 carbon alkyl side chains and combinations thereof. In particular, the block copolymer can include an ABC triblock structure having a polybutylacrylate segment, a polyethylacrylate segment, and a polymethacrylic acid segment, for example..

The contrast agent having functionality different than the first functionality of the nanospecies can include, but is not limited to, magnetic compounds, paramagnetic compounds, radioisotopes, fluorescent compounds, organic dyes, and combinations thereof. The magnetic compounds, and paramagnetic compounds are described above in regard to nanospecies. It should be noted that the nanospecies is a quantum dot, while the contrast agent is not a quantum dot. The semiconductor, metal, and metal oxide nanoparticles, metalloid and metalloid oxide nanoparticles, the lanthanide series metal nanoparticles, magnetic compounds, magnetic compounds, paramagnetic compounds, and radioisotopes, can each be used as a contrast agent. The particular configuration depends, at least in part, upon the application and detection system(s) used. The amount of contrast agent disposed on the nanospecies depends, at least in part, upon the strength of signal desired, the layers, if any, disposed on the nanospecies, and the imaging detection system. In addition, the amount of contrast agent disposed on the nanospecies should not interfere with the desired signal from the nanospecies or other chemical properties.

The radioisotopes can include, but are not limited to, indium (¹¹¹In), yttrium (⁹⁰Y), ⁹⁹Tc (technetium), ²⁰¹Tl (thallium), ⁶⁷Ga (gallium), ¹²³I (iodine), ¹³¹I (iodine), ³²P (phosphorus), ¹⁵³Sm (samarium), ¹⁸⁶Re, ¹⁸⁸Re (rhenium), ¹⁶⁵Dy (dysprosium), ¹⁶⁶Ho (holmium), and combinations thereof.

The fluorescent compounds (*e.g*., organic dyes) can include, but are not limited to, fluorescein, Pyrene, Coumarin, BODIPY, Oregon green, Rhodamine, and Texas Red, or cyanine dyes. Additional dyes can be found from commercial websites, such as Molecular Probes (www.probes.com).

The fluorescent compounds can include, but are not limited to, ,1,5 IAEDANS; 1,8-ANS; 4-Methylumbelliferone; 5-carboxy-2,7-dichlorofluorescein; 5-carboxyfluorescein (5-FAM); 5-Carboxynapthofluorescein; 5-carboxytetramethylrhodamine (5-TAMRA); 5-FAM (5-carboxyfluorescein); 5-HAT (hydroxy tryptamine); 5-hydroxy tryptamine (HAT); 5-ROX (carboxy-X-rhodamine); 5-TAMRA (5-carboxytetramethylrhodamine); 6-carboxyrhodamine 6G; 6-CR 6G; 6-JOE; 7-amino-4-methylcoumarin; 7-aminoactinomycin D (7-AAD); 7-hydroxy-4-methylcoumarin; 9-amino-6-chloro-2-methoxyacridine; ABQ; Acid Fuchsin; ACMA (9-amino-6-chloro-2-methoxyacridine); Acridine Orange; Acridine Red; Acridine Yellow; Acriflavin; Acriflavin Feulgen SITSA; Aequorin (Photoprotein); AFPs - AutoFluorescent Protein - (Quantum Biotechnologies); Alexa Fluor 350™; Alexa Fluor 430™; Alexa Fluor 488T™; Alexa Fluor 532™; Alexa Fluor 546™; Alexa Fluor 568™; Alexa Fluor 594™; Alexa Fluor 633™; Alexa Fluor 647™; Alexa Fluor 660™; Alexa Fluor 680™; Alizarin Complexon; Alizarin Red; Allophycocyanin (APC); AMC, AMCA-S; AMCA (Aminomethylcoumarin); AMCA-X; Aminoactinomycin D; Aminocoumarin; Aminomethylcoumarin (AMCA); Anilin Blue; Anthrocyl stearate; APC (Allophycocyanin); APC-Cy7; APTRA-BTC; APTS; Astrazon Brilliant Red 4G; Astrazon Orange R; Astrazon Red 6B; Astrazon Yellow 7 GLL; Atabrine; ATTO-TAG™ CBQCA; ATTO-TAG™ FQ; Auramine; Aurophosphine G; Aurophosphine; BAO 9 (Bisaminophenyloxadiazole); BCECF (high pH); BCECF (low pH); Berberine Sulphate; Beta Lactamase; Bimane; Bisbenzamide; Bisbenzimide (Hoechst); bis-BTC; Blancophor FFG; Blancophor SV; BOBO™ -1; BOBO™ -3; Bodipy 492/515; Bodipy 493/503; Bodipy 500/510; Bodipy 505/515; Bodipy 530/550; Bodipy 542/563; Bodipy 558/568; Bodipy 564/570; Bodipy 576/589; Bodipy 581/591; Bodipy 630/650-X; Bodipy 650/665-X; Bodipy 665/676; Bodipy Fl; Bodipy FL ATP; Bodipy Fl-Ceramide; Bodipy R6G SE; Bodipy TMR; Bodipy TMR-X conjugate; Bodipy TMR-X, SE; Bodipy TR; Bodipy TR ATP; Bodipy TR-X SE; BO-PRO™ -1; BO-PRO™ -3; Brilliant Sulphoflavin FF; BTC; BTC-5N; Calcein; Calcein Blue; Calcium Crimson™; Calcium Green; Calcium Green-1 Ca²⁺ Dye; Calcium Green-2 Ca²⁺; Calcium Green-5N Ca²⁺; Calcium Green-C18 Ca²⁺; Calcium Orange; Calcofluor White; Carboxy-X-rhodamine (5-ROX); Cascade Blue™; Cascade Yellow; Catecholamine; CCF2 (GeneBlazer); CFDA; chlorophyll; chromomycin A; chromomycin A; CL-NERF; CMFDA; Coumarin Phalloidin; C-phycocyanine; CPM Methylcoumarin; CTC; CTC Formazan; Cy2™; Cy3.1 8; Cy3.5™; Cy3™; Cy5.1 8; Cy5.5™; Cy5™; Cy7™; cyclic AMP Fluorosensor (FiCRhR); Dabcyl; Dansyl; Dansyl Amine; Dansyl Cadaverine; Dansyl Chloride; Dansyl DHPE; Dansyl fluoride; DAPI; Dapoxyl; Dapoxyl 2; Dapoxyl 3' DCFDA; DCFH (dichlorodihydrofluorescein diacetate); DDAO; DHR (dihydrohodamine 123); Di-4-ANEPPS; Di-8-ANEPPS (non-ratio); DiA (4-Di-16-ASP); dichlorodihydrofluorescein Diacetate (DCFH); DiD - Lipophilic Tracer; DiD (DiIC18(5)); DIDS; Dihydorhodamine 123 (DHR); DiI (DiIC18(3)); Dinitrophenol; DiO (DiOC18(3)); DiR; DiR (DiIC18(7)); DM-NERF (high pH); DNP; dopamine; DTAF; DY-630-NHS; DY-635-NHS; ELF 97; Eosin; Erythrosin; Erythrosin ITC; ethidium bromide; ethidium homodimer -1 (EthD-1); Euchrysin; EukoLight; Europium (III) chloride; EYFP; Fast Blue; FDA; Feulgen (Pararosaniline); FIF (Formaldehyde-Induced Fluorescence); FITC; Flazo Orange; Fluo-3; Fluo-4; Fluorescein (FITC); Fluorescein Diacetate; Fluoro-Emerald; Fluoro-Gold (Hydroxystilbamidine); Fluor-Ruby; FluorX; FM 1-43™; FM 4-46; Fura Red™ (high pH); Fura Red™/Fluo-3; Fura-2; Fura-2BCECF; Genacryl Brilliant Red B; Genacryl Brilliant Yellow 10GF; Genacryl Pink 3G; Genacryl Yellow 5GF; GeneBlazer (CCF2); Gloxalic Acid; Granular blue; Haematoporphyrin; Hoechst 33258; Hoechst 33342; Hoechst 34580; HPTS; Hydroxycoumarin; Hydroxystilbamidine (FluoroGold); Hydroxytryptamine; Indo-1, high calcium; Indo-1, low calcium; Indodicarbocyanine (DiD); Indotricarbocyanine (DiR); Intrawhite Cf; JC-1; JO-JO-1; JO-PRO-1; LaserPro; Laurodan; LDS 751 (DNA); LDS 751 (RNA); Leucophor PAF; Leucophor SF; Leucophor WS; Lissamine Rhodamine; Lissamine Rhodamine B; Calcein/Ethidium homodimer; LOLO-1; LO-PRO-1; Lucifer Yellow; Lyso Tracker Blue; Lyso Tracker Blue-White; Lyso Tracker Green; Lyso Tracker Red; Lyso Tracker Yellow; LysoSensor Blue; LysoSensor Green; LysoSensor Yellow/Blue; Mag Green; Magdala Red (Phloxin B); Mag-Fura Red; Mag-Fura-2; Mag-Fura-5; Mag-Indo-1; Magnesium Green; Magnesium Orange; Malachite Green; Marina Blue; Maxilon Brilliant Flavin 10 GFF; Maxilon Brilliant Flavin 8 GFF; Merocyanin; Methoxycoumarin; Mitotracker Green FM; Mitotracker Orange; Mitotracker Red; Mitramycin; Monobromobimane; Monobromobimane (mBBr-GSH); Monochlorobimane; MPS (Methyl Green Pyronine Stilbene); NBD; NBD Amine; Nile Red; Nitrobenzoxadidole; Noradrenaline; Nuclear Fast Red; Nuclear Yellow; Nylosan Brilliant Iavin E8G; Oregon Green; Oregon Green 488-X; Oregon Green™; Oregon Green™ 488; Oregon Green™ 500; Oregon Green™ 514; Pacific Blue; Pararosaniline (Feulgen); PBFI; PE-Cy5; PE-Cy7; PerCP; PerCP-Cy5.5; PE-TexasRed [Red 613]; Phloxin B (Magdala Red); Phorwite AR; Phorwite BKL; Phorwite Rev; Phorwite RPA; Phosphine 3R; PhotoResist; Phycoerythrin B [PE]; Phycoerythrin R [PE]; PKH26 (Sigma); PKH67; PMIA; Pontochrome Blue Black; POPO-1; POPO-3; PO-PRO-1; PO-PRO-3; Primuline; Procion Yellow; Propidium Iodid (PI); PyMPO; Pyrene; Pyronine ; Pyronine B; Pyrozal Brilliant Flavin 7GF; QSY 7; Quinacrine Mustard; Red 613 [PE-TexasRed]; Resorufin; RH 414; Rhod-2; Rhodamine; Rhodamine 110; Rhodamine 123; Rhodamine 5 GLD; Rhodamine 6G; Rhodamine B; Rhodamine B 200; Rhodamine B extra; Rhodamine BB; Rhodamine BG; Rhodamine Green; Rhodamine Phallicidine; Rhodamine Phalloidine; Rhodamine Red; Rhodamine WT; Rose Bengal; R-phycocyanine; R-phycoerythrin (PE); S65A; S65C; S65L; S65T; SBFI; Serotonin; Sevron Brilliant Red 2B; Sevron Brilliant Red 4G; Sevron Brilliant Red B; Sevron Orange; Sevron Yellow L; SITS; SITS (Primuline); SITS (stilbene isothiosulphonic acid); SNAFL calcein; SNAFL-1; SNAFL-2; SNARF calcein; SNARF1; Sodium Green; SpectrumAqua; SpectrumGreen; SpectrumOrange; Spectrum Red; SPQ (6-methoxy-N-(3-sulfopropyl)quinolinium); Stilbene; Sulphorhodamine B can C; Sulphorhodamine Extra; SYTO 11; SYTO 12; SYTO 13; SYTO 14; SYTO 15; SYTO 16; SYTO 17; SYTO 18; SYTO 20; SYTO 21; SYTO 22; SYTO 23; SYTO 24; SYTO 25; SYTO 40; SYTO 41; SYTO 42; SYTO 43; SYTO 44; SYTO 45; SYTO 59; SYTO 60; SYTO 61; SYTO 62; SYTO 63; SYTO 64; SYTO 80; SYTO 81; SYTO 82; SYTO 83; SYTO 84; SYTO 85; SYTOX Blue; SYTOX Green; SYTOX Orange; Tetracycline; Tetramethylrhodamine (TRITC); Texas Red™; Texas Red-X™ conjugate; Thiadicarbocyanine (DiSC3); Thiazine Red R; Thiazole Orange; Thioflavin 5; Thioflavin S; Thioflavin TCN; Thiolyte; Thiozole Orange; Tinopol CBS (Calcofluor White); TMR; TO-PRO-1; TO-PRO-3; TO-PRO-5; TOTO-1; TOTO-3; Tricolor (PE-Cy5); TRITC TetramethylRodamineIsoThioCyanate; True Blue; TruRed; Ultralite; Uranine B; Uvitex SFC; WW 781; X-Rhodamine; XRITC; Xylene Orange; Y66F; Y66H; Y66W; YO-PRO-1; YO-PRO-3; YOYO-1;YOYO-3, Sybr Green, Thiazole orange (interchelating dyes), or combinations thereof.

As mentioned above, the nanostructure can be attached to a probe molecule. The probe molecule can be any molecule capable of being linked to the nanostructure either directly or indirectly via one or more linkers. The probe molecule can be attached by any stable physical or chemical association to the nanostructure directly or indirectly by any suitable means.

In one embodiment, the probe molecule has an affinity for one or more target molecules (*e.g*., cancer cell) for which detection (*e.g*., determining the presence of and/or proximal position within the vessel (body)) is desired. If, for example, the target molecule is a nucleic acid sequence, the probe molecule should be chosen so as to be substantially complementary to the target molecule sequence, such that the hybridization of the target and the probe occurs. The term "substantially complementary," means that the probe molecules are sufficiently complementary to the target sequences to hybridize under the selected reaction conditions.

The probe molecule and the target molecule can include, but are not limited to, polypeptides (*e.g*., protein such as, but not limited to an antibody (monoclonal or polyclonal)), nucleic acids (both monomeric and oligomeric), polysaccharides, sugars, fatty acids, steroids, purines, pyrimidines, drugs (*e.g*., small compound drugs), ligands, or combinations thereof.

Use of the phrase "polypeptide" or "protein" is intended to encompass a protein, a glycoprotein, a polypeptide, a peptide, and the like, whether isolated from nature, of viral, bacterial, plant, or animal (*e.g*., mammalian, such as human) origin, or synthetic, and fragments thereof. A preferred protein or fragment thereof includes, but is not limited to, an antigen, an epitope of an antigen, an antibody, or an antigenically reactive fragment of an antibody.

Use of the phrase "nucleic acid" is intended to encompass DNA and RNA, whether isolated from nature, of viral, bacterial, plant or animal (*e.g*., mammalian, such as a human) origin, synthetic, single-stranded, double-stranded, comprising naturally or non-naturally occurring nucleotides, or chemically modified.

In addition, the probe can also include, but is not limited to, a drug, a therapeutic agent, radiological agent, a small molecule drug, and combinations thereof, that can be used to treat the target molecule and/or the associated disease and condition of interest. The drug, therapeutic agent, and radiological agent can be selected based on the intended treatment as well as the condition and/or disease to be treated. In an embodiment, the nanostructure can include two or more probes used to treat a condition and/or disease.

The following is a nonlimiting illustrative list of probes that can be used: Proleukin^{™}, Campath^{™}, Panretin^{™}, Zyloprim^{™}, Hexalen^{™}, Ethyol^{™}, Arimidex^{™}, Trisenox^{™}, Elspar^{™}, TICE BCG^{™}, Targretin^{™}, Blenoxane^{™}, Busulfex^{™}, Myleran^{™}, Methosarb^{™}, Xeloda^{™}, Paraplatin^{™}, BCNU, BiCNU^{™}, Gliadel Wafer^{™}; Celebrex^{™}, Leukeran^{™}, Platinol^{™}, Leustatin,-2-CdA^{™}, Cytoxan, Neosar^{™}, Cytoxan Injection^{™}, Cytoxan Tablet^{™}, Cytosar-U^{™}, DepoCyt^{™}, DTIC-Dome^{™}, Cosmegen^{™}, Aranesp^{™}, DanuoXome^{™}, Daunorubicin^{™}, Cerubidine^{™}, Ontak^{™}, Zinecard^{™}, Taxotere^{™}, Adriamycin, Rubex^{™}, Adriamycin PFS Injectionintravenous Injection^{™}, Doxil^{™}, Dromostanolone^{™}, Masterone^{™}, Elliott's B Solution^{™}, Ellence^{™}, epogen^{™}, Emcyt^{™}, Etopophos^{™}, Vepesid^{™}, Aromasin^{™}, Neupogen^{™}, FUDR^{™}, Fludara^{™}, Adrucil^{™}, Faslodex^{™}, Gemzar^{™}, Mylotarg^{™}, Zoladex Implant^{™}, Zoladrex^{™}, Hydrea^{™}, Zevalin^{™}, Idamycin^{™}, IFEX^{™}, Gleevec^{™}, Roferon-A^{™}, Intron A^{™}, Camptosar^{™}, Femara^{™}, Wellcovorin, Leucovorin^{™}, Leucovorin^{™}, Ergamisol^{™}, CeeBU^{™}, Mustargen^{™}, Megace^{™}, Alkeran^{™}, Purinethol^{™}, Mesnex^{™}, Methotrexate^{™}; Uvadex^{™}, Mutamycin^{™}, Mitozytrex^{™}, Lysodren^{™}, Novatrone^{™}, Durabolin-50^{™}, Verluma^{™}, Neumega^{™}, Eloxatin^{™}, Paxene^{™}, Taxol^{™}, Aredia^{™}, Adagen (Pegademase Bovine)^{™}, Oncaspar^{™}, Neulasta^{™}, Nipent^{™}, Vercyte^{™}, Mithracin^{™}, Photofrin^{™}, Matulane^{™}, Atabrine^{™}, Elitek^{™}, Rituxan^{™}, Prokine^{™}, Zanosar^{™}, Sclerosol^{™}, Nolvadex^{™}, Temodar^{™}, Vumon^{™}, Teslac^{™}, Thioguanine^{™}, Thioplex^{™}, Hycamtin^{™}, Fareston^{™}, Bexxar^{™}, Herceptin^{™}, Vesanoid^{™}, Uracil Mustard Capsules^{™}, Valstar^{™}, Velban^{™}, Oncovin^{™}, Navelbine^{™}, and Zometa^{™}.

In an embodiment, the nanostructure can include at least two different types of probes, one being a targeting probe that targets certain cells or compounds associated with a condition and/or disease, while the second probe is a drug used to treat the disease. In this manner, the nanostructure acts as a detection component, a delivery component to the cells of interest, and a delivery component for the treatment agent. The detection of the nanospecies can be used to ensure the delivery of the nanostructure to its intended destination as well as the quantity of nanostructures delivered to the destination.

FIG. 1 illustrates an embodiment of a multimodality nanostructure 10a. The multimodality nanostructure 10a includes, but is not limited to, a quantum dot 12, a polyamine layer 14, a chelator 18, a contrast agent 22, and a bio-compatibility agent or compound 24. The chelator 18 is attached to the polyamine layer 14 via a linker 16 (*e.g*., an amine function group of the polyamine layer 14). In addition, the bio-compatibility compound 24 is attached to the polyamine layer 14 via the linker 16. In another embodiment, the linker for the chelator 18 and the bio-compatibility compound 24 are different. In another embodiment, the multimodality nanostructure 10a can include two or more contrast agents with different functionalities.

For example, a specific example would include a QD coated with polylysine, where PEG-COOH and DTPA are conjugated to the amines, and a paramagnetic and/or an isotopic compound is chelated by the DTPA.

FIG. 2 illustrates an embodiment of a multimodality nanostructure 10b. The multimodality nanostructure 10b includes, but is not limited to, a quantum dot 12, a capping ligand 32, a polymer layer 34, a chelator 18, a contrast agent 22, and a bio-compatibility compound 24. The chelator 18 is attached to the polymer layer 34 via a linker 16. In addition, the bio-compatibility compound 24 is attached to the polymer layer 34 via the linker 16. In another embodiment, the linker for the chelator 18 and the bio-compatibility compound 24 are different. In another embodiment, the multimodality nanostructure 10b can include two or more contrast agents with different functionalities.

For example, a specific example would be include a hydrophobic ligand protected QD that is overcoated with a layer of amphiphilic polymer, where the PEG and DTPA are conjugated to the amphiphilic polymer. A paramagnetic and/or an isotopic compound is chelated by the DTPA. Another example includes a hydrophobic ligand protected QD that is overcoated with a mixture of lipid-PEG and lipid-DTPA. A paramagnetic and/or an isotopic compound is chelated by the DTPA.

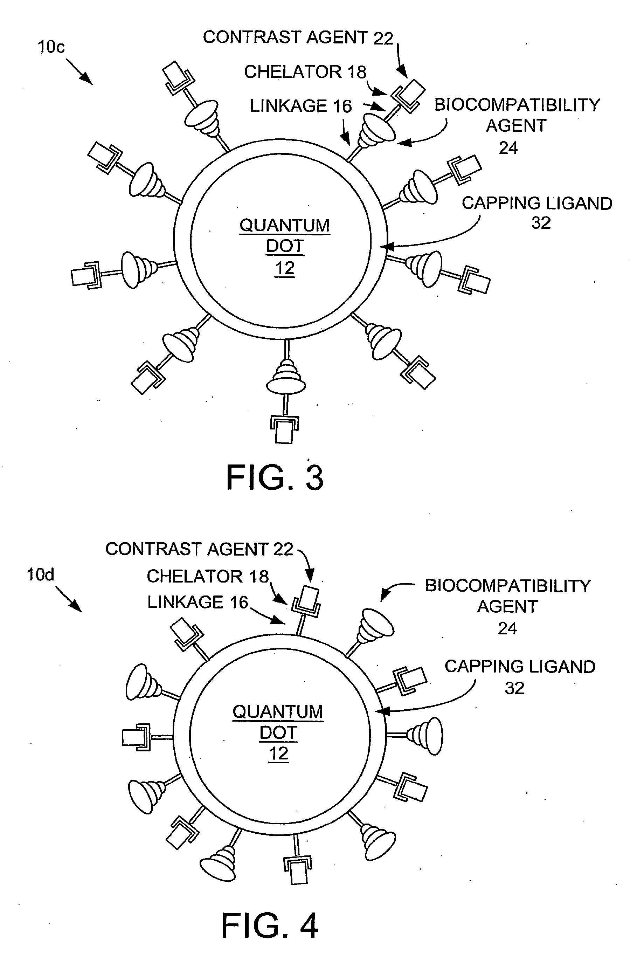

FIG. 3 illustrates an embodiment of a multimodality nanostructure 10c. The multimodality nanostructure 10c includes; but is not limited to, a quantum dot 12, a capping ligand 32, a chelator 18, a contrast agent 22, and a bio-compatibility compound 24. The bio-compatibility compound 24 is attached to the capping ligand 32 via the linker 16. The chelator 18 is attached via the linker 16 to the bio-compatibility compound 24 (*e.g*., a PEG compound with having an amine function group). In another embodiment, the linker for the chelator 18 and the bio-34 via the linker 16. In another embodiment, the linker for the dye 38 and the bio-compatibility compound 24 are different. In another embodiment, the multimodality nanostructure 10f can include two or more contrast agents with different functionalities.

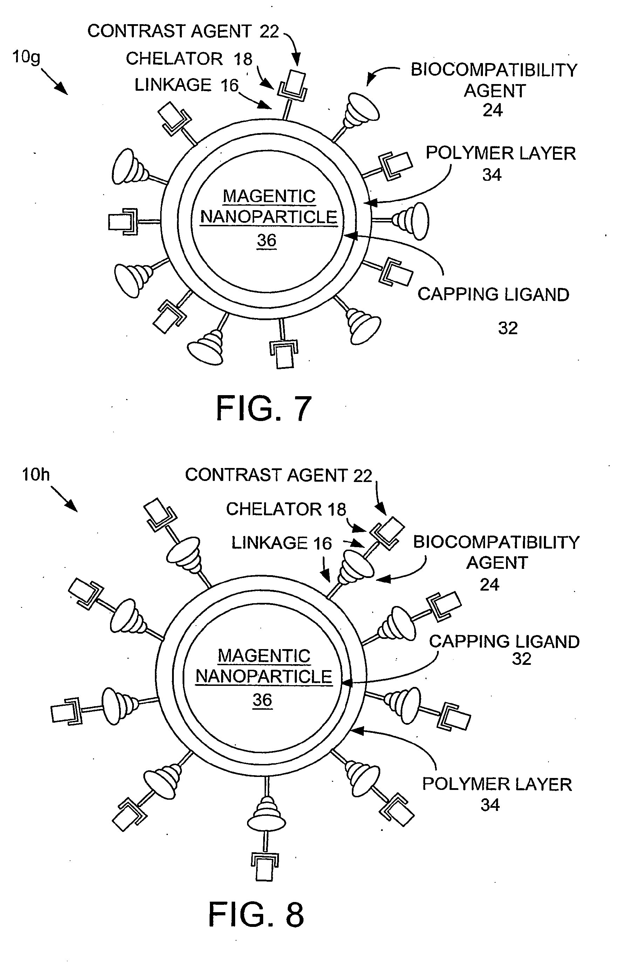

FIG. 7 illustrates a multimodality nanostructure 10g. The multimodality nanostructure 10g includes, but is not limited to, a magnetic nanoparticle 36 (in another embodiment a paramagnetic nanoparticle), a capping ligand 32, a polymer layer 34, a chelator 18, a contrast agent 22 (*e.g*., a radioactive compound), and a bio-compatibility compound 24. The chelator 18 is attached to the polymer layer 34 via a linker 16. In addition, the bio-compatibility compound 24 is attached to the polymer layer 34 via the linker 16. In another embodiment, the linker for the chelator 18 and the bio-compatibility compound 24 are different. In another embodiment, the multimodality nanostructures 10g can include two or more contrast agents with different functionalities.

FIG. 8 illustrates a multimodality nanostructure 10h. The multimodality nanostructure 10h includes, but is not limited to, a magnetic nanoparticle 36 (in another embodiment a paramagnetic nanoparticle), a capping ligand 32, a polymer layer 34, a chelator 18, a contrast agent 22, and a bio-compatibility compound 24. The bio-compatibility compound 24 is attached to the capping ligand 32 via the linker 16. The chelator 18 is attached via the linker 16 to the bio-compatibility compound 24 (*e.g*., a PEG compound with having an amine function group). In another embodiment, the linker for the chelator 18 and the bio-compatibility compound 24 are different. In another embodiment, the multimodality nanostructure 10h can include two or more contrast agents with different functionalities.

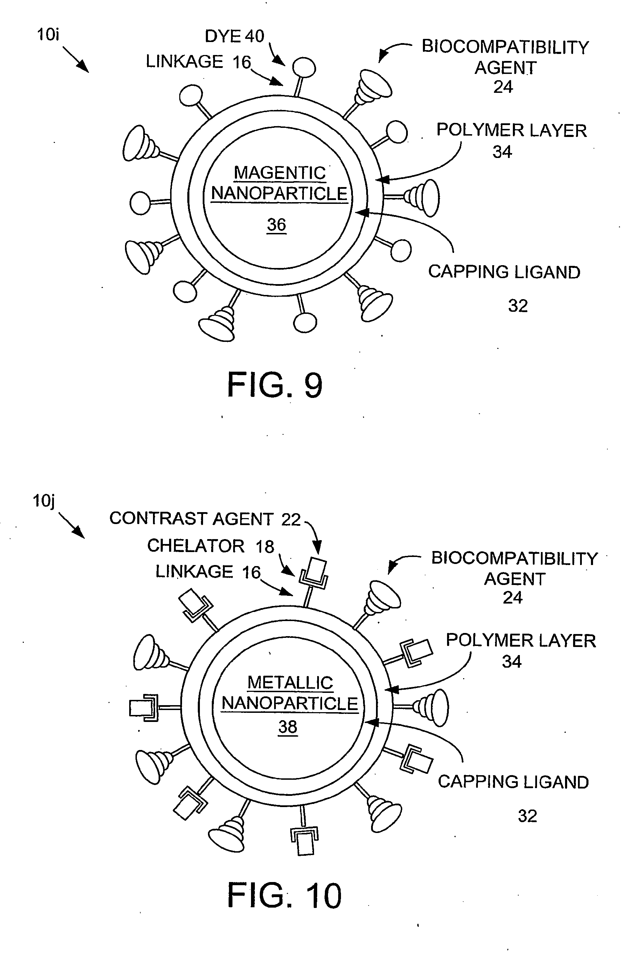

FIG. 9 illustrates a multimodality nanostructure 10i. The multimodality nanostructure 10i includes, but is not limited to, a magnetic nanoparticle 36 (in another embodiment a paramagnetic nanoparticle), a capping ligand 32, a polymer layer 34, a dye 38, and a bio-compatibility compound 24. The dye 38 is attached to the polymer layer 34 via a linker 16 and in another embodiment, the dye 38 is absorbed onto the polymer layer 34. In addition, the bio-compatibility compound 24 is attached to the polymer layer 34 via the linker 16. In another embodiment, the linker for the dye 38 and the bio-compatibility compound 24 are compatibility compound 24 are different. In another embodiment, the multimodality nanostructure 10c can include two or more contrast agents with different functionalities.

For example, a QD is coated with PEG modified polylysine, where the other termini of the PEG achelating reagent is conjugated (*e.g*., DTPA, EDTA, and NTA). A paramagnetic or an isotopic compound is chelated by the chelating compound.

FIG. 4 illustrates an embodiment of a multimodality nanostructure 10d. The multimodality nanostructure 10d includes, but is not limited to, a quantum dot 12, a capping layer 32, a chelator 18, a contrast agent 22, and a bio-compatibility compound 24. The chelator 18 is attached to the capping layer 32 via a linker 16. In addition, the bio-compatibility compound 24 is attached to the capping layer 32 via the linker 16. In another embodiment, the linker for the chelator 18 and the bio-compatibility compound 24 are different. In another embodiment, the multimodality nanostructure 10d can include two or more contrast agents with different. functionalities.

For example, an amine-modified DTPA or NTA and thio- or amine-modified PEG co-adsorbed on the surface of a QD. A paramagnetic or an isotopic compound is chelated by the chelating reagent

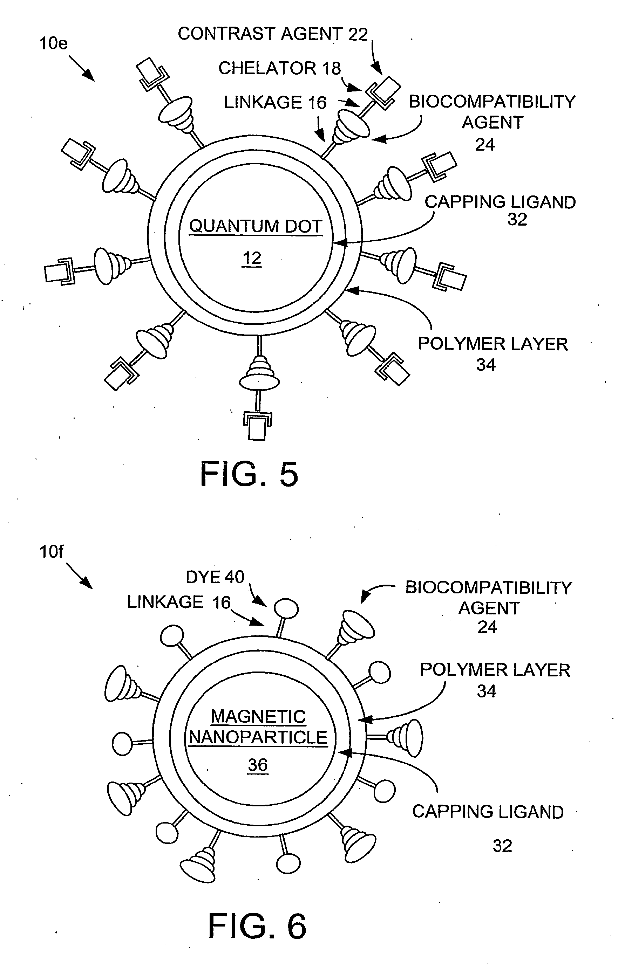

FIG. 5 illustrates an embodiment of a multimodality nanostructure 10e. The multimodality nanostructure 10e includes, but is not limited to, a quantum dot 12, a capping ligand 32, a polymer layer 34, a chelator 18, a contrast agent 22, and a bio-compatibility compound 24. The bio-compatibility compound 24 is attached to the capping ligand 32 via the linker 16. The chelator 18 is attached via the linker 16 to the bio-compatibility compound 24 (*e.g*., a PEG compound with having an amine function group). In another embodiment, the linker for the chelator 18 and the bio-compatibility compound 24 are different. In another embodiment, the multimodality nanostructure 10e can include two or more contrast agents with different functionalities.

FIG. 6 illustrates a multimodality nanostructure 10f. The multimodality nanostructure 10f includes, but is not limited to, a magnetic nanoparticle, a capping ligand 32, a polymer layer 34, a dye 38, and a bio-compatibility compound 24. The dye 38 is attached to the polymer layer 34 via a linker 16 and in another embodiment, the dye 38 is absorbed onto the polymer layer 34. In addition, the bio-compatibility compound 24 is attached to the polymer layer different. In another embodiment, the multimodality nanostructure 10i can include two or more contrast agents with different functionalities.

FIG. 10 illustrates a multimodality nanostructure 10j. The multimodality nanostructure 10j includes, but is not limited to, a metallic nanoparticle 38, a capping ligand 32, a polymer layer 34, a chelator 18, a contrast agent 22, and a bio-compatibility compound 24. The chelator 18 is attached to the polymer layer 34 via a linker 16. In addition, the bio-compatibility compound 24 is attached to the polymer layer 34 via the linker 16. In another embodiment, the linker for the chelator 18 and the bio-compatibility compound 24 are different. In another embodiment, the multimodality nanostructure 10j can include two or more contrast agents with different functionalities.

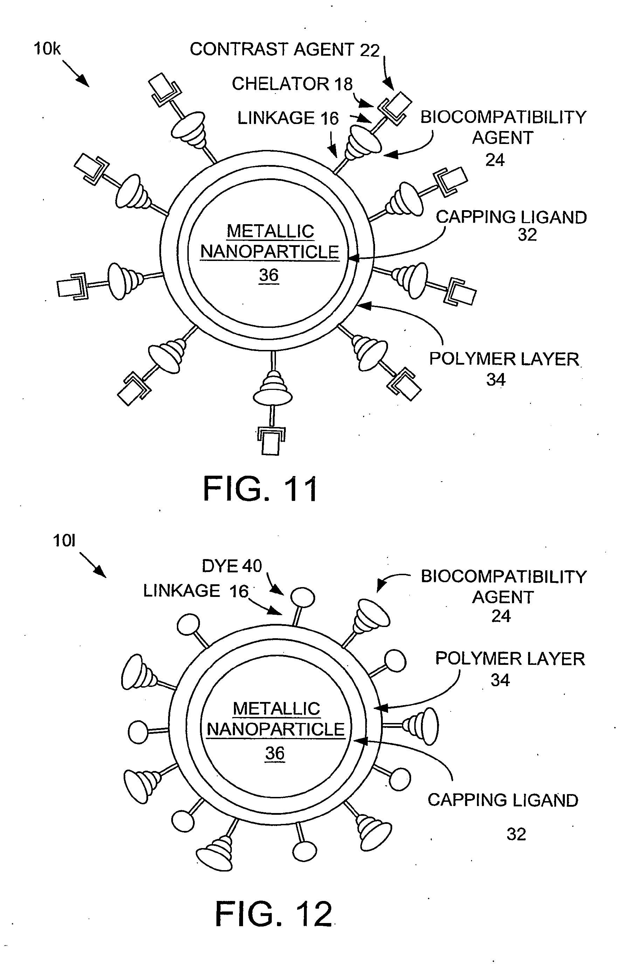

FIG. 11 illustrates a multimodality nanostructure 10k. The multimodality nanostructure 10k includes, but is not limited to, a metallic nanoparticle 38, a capping ligand 32, a polymer layer 34, a chelator 18, a contrast agent 22, and a bio-compatibility compound 24. The bio-compatibility compound 24 is attached to the capping ligand 32 via the linker 16. The chelator 18 is attached via the linker 16 to the bio-compatibility compound 24 (*e.g*., a PEG compound with having an amine function group). In another embodiment, the linker for the chelator 18 and the bio-compatibility compound 24 are different. In another embodiment, the multimodality nanostructure 10k can include two or more contrast agents with different functionalities.

FIG. 12 illustrates a multimodality nanostructure 101. The multimodality nanostructure 101 includes, but is not limited to, a metallic nanoparticle 38, a capping ligand 32, a polymer layer 34, a dye 38, and a bio-compatibility compound 24. The dye 38 is attached to the polymer layer 34 via a linker 16 and in another embodiment, the dye 38 is absorbed onto the polymer layer 34. In addition, the bio-compatibility compound 24 is attached to the polymer layer 34 via the linker 16. In another embodiment, the linker for the dye 38 and the bio-compatibility compound 24 are different In another embodiment, the multimodality nanostructure 101 can include two or more contrast agents with different functionalities.

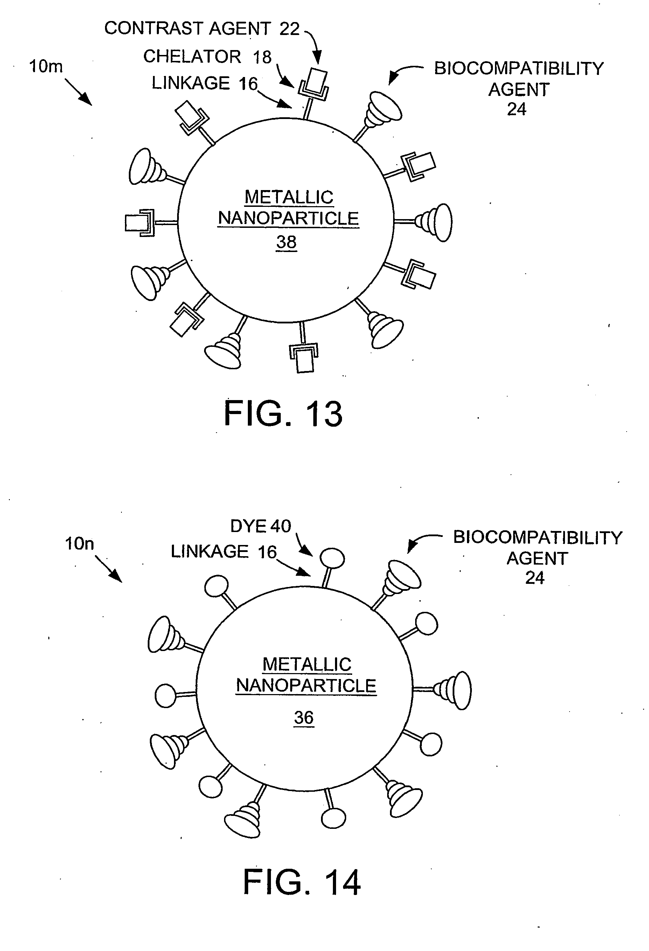

FIG. 13 illustrates a multimodality nanostructure 10m. The multimodality nanostructure 10m includes, but is not limited to, a metallic nanoparticle 38, a chelator 18, a contrast agent 22, and a bio-compatibility compound 24. The chelator 18 is attached to the metallic nanoparticle 38 via a linker 16. In addition, the bio-compatibility compound 24 is attached to the metallic nanoparticle 38 via the linker 16. In another embodiment, the linker for the chelator 18 and the bio-compatibility compound 24 are different. In another embodiment, the multimodality nanostructure 10m can include two or more contrast agents with different functionalities.

FIG. 14 illustrates a multimodality nanostructure 10n. The multimodality nanostructure 10n includes, but is not limited to, a metallic nanoparticle 38, a dye 38, and a bio-compatibility compound 24. The dye 38 is attached to the metallic nanoparticle 38 via a linker 16. In addition, the bio-compatibility compound 24 is attached to the metallic nanoparticle 38 via the linker 16. In another embodiment, the linker for the chelator 18 and the bio-compatibility compound 24 are different. In another embodiment, the multimodality nanostructure 10n can include two or more contrast agents with different functionalities.

### Examples

Now having described the embodiments of the nanostructure in general, example 1-12 describes some embodiments of the nanostructure. While embodiments of nanostructures are described in connection with examples 1-12 and the corresponding text and figures, there is no intent to limit embodiments of the nanospecies to these descriptions. On the contrary, the intent is to cover all alternatives, modifications, and equivalents included within the spirit and scope of embodiments of the present disclosure.

### Example 1: A Gd-Quantum Dot (QD) nanostructure.

PEG and Gd-DTPA graphed polylysine can be prepared using literature procedures with some modifications (*e.g*., by changing the PEG and DTPA substitution degree) (See, Review of Scientific Instruments (2003) 74, 4158-4163; Biophysical Journal 75, 1998, 2163-2169; Review of Scientific Instruments (2003) 74, 4012-4020; and Hypertension 2001, 38, 1158-1161, which are included herein by reference). It should be noted that the chelating reagents are not limited to DTPA could include DOPA, EDTA, and the like). The PEG and Gd-DTPA graphed polylysine can be titrated into QD-mercaptoacetic acid solution at a controlled ratio (*i.e*., until the solution become optically clear and essentially aggregates free examined by fluorescence microscopy). The multiple amine groups quickly adsorb onto QD surface whereas PEG chains and Gd-DTPA each face outward to the solution. The resulting nanostructure has dual fluorescent and magnetic functionalities.

### Example 2: A Gd-QD nanostructure

To prepare lipid-DTPA-Gd coated QDs, DSPE-DTPA and DSPE-mPEG are mixed in a controlled ratio and are dissolved in a basic solution (above about a pH of 7). QDs are purified and suspended in dichloromethane. The two solutions are mixed together and sonicated with a probe sonicator. After formation of a optically clear QD solution and removal of the excess lipid ligands, the pH of the QD solution is adjusted to 5 using an acid (*e.g*., HCl), followed by addition of GdCl₃-6H₂O. The Gd³⁺-labeled QDs are purified with a filtration column. The resulting nanostructure also has dual fluorescent and magnetic properties.

### Example 2.1

To prepare lipid-DTPA-Gd coated QDs, Gd-QD DSPE-DTPA and DSPE-mPEG are mixed in a controlled ratio in chloroform and added to QD-chloroform solution. After slow vacuum drying, water or aqueous buffer are added to the lipid and particle dry film. Agitation or sonication lead to formation of an optically clear QD solution. After removal of the excess lipid ligands, the pH of the QD solution is adjusted to 5 using an acid (*e.g*., HCl), followed by addition of GdCl₃·6H₂O. The Gd³⁺-labeled QDs are purified with a filtration column. The resulting nanostructure also has dual fluorescent and magnetic properties.

### Example 3: A Gd-QD nanostructure

To prepare DTPA-Gd coated QDs, DSPE-PEG-NH₂ are modified at the NH₂ end with DTPA using EDAC as a crosslinker, or using DTPA anhydride. The purified product is dissolved in a basic solution (above about a pH of 7). The QDs are purified and suspended in dichloromethane. The two solutions are mixed together and sonicated with a probe sonicator. After formation of an optically clear QD solution and removal of the excess lipid ligands, the pH of the QD solution is adjusted to about 5 using an acid (*e.g*., HCl), followed by addition of GdCl₃·6H₂O. The Gd³⁺-labeled QDs are purified with a filtration column. The resulting nanostructure also has dual fluorescent and magnetic properties.

### Example 3.1 Gd-QD

To prepare DTPA-Gd coated QDs, DSPE-PEG-NH₂ are mixed QDs in chloroform solution. After slow vacuum drying, water or aqueous buffer are added to the lipid and particle dry film. Agitation or sonication lead to formation of an optically clear QD solution. After removal of the excess lipid ligands, diethylenetriaminepentaacetic acid dianhydride is used to modify free amine groups into DTPA. Excess (unbound) DTPA are purified out by filtration columns or dialysis. The pH of the QD solution is then adjusted to 5 using an acid (*e.g.*, HCl), followed by addition of GdCl₃·6H₂O. The Gd³⁺-labeled dots are purified with a filtration column. The resulting nanostructure also has dual fluorescent and magnetic properties.

### Example 4: A Gd-QD nanostructure

To prepare DTPA-Gd coated QDs, amphiphilic polymer poly(maleic anhydride alt-1-tetradecene) is first react with diamines, such as 2,2'-(ethylenedioxy)diethylamine, then modified into DTPA using diethylenetriaminepentaacetic acid dianhydride. To solublize the QDs, the purified polymer can be either mixed with QDs in organic solvents (*e.g*., chloroform, ethanol, or solvent mixtures) then dried and suspended into aqueous solution, or the purified polymer can be suspended in aqueous solution, after addition of QDs in dichloromethane and sonication, the dots are suspended in aqueous solution. The polymer coated QDs are then mixed with the Gd³⁺ solution and purified with filtration column or centrifugation. The resulting nanostructure also has dual fluorescent and magnetic properties.

### Example 5: ⁶⁷Gd, ¹¹¹In or ⁹⁰Y QD nanostructure

Based on similar procedures described above (the DTPA coated QDs are described in example 1-4, except Gd³⁺ is added), ⁶⁷Gd, ¹¹¹In, ⁹⁰Y and other isotopic agents such as those described above can be chelated to the QD surface, leading to radioactive-fluorescent probes.

### Example 6: ¹¹¹In or ⁹⁰Y labeled magnetic nanoparticle nanostructure

The hydrophobic magnetic nanoparticles are prepared as described in the literature (See, J. Am. Chem. Soc. 2001, 123, 12798-12801; Chem Commun., 2003, 927-934; J. Am. Chem. Soc. 2004, 126, 273-279; Nano Lett. 2004 4(1); 187-190; J. Am. Chem. Soc. 2002; 124, 8204-8205; and J. Phys. Chem. B. ; 2001; 105, 7913-7919). The ligand and polymer coating procedures are described in Example 1-4. ⁶⁷Gd, ¹¹¹In, and ⁹⁰Y, *etc* can be chelated to the surface of magnetic nanoparticlesm leading to magnetic-radioactive dual probes.

### Example 7: ¹¹¹In or ⁹⁰Y and Gd³⁺ labeled QD nanostructure

¹¹¹In, ⁹⁰Y, Gd³⁺ can be co-chelated to DTPA or DOTA functionalized QDs, leading to triple-modality probes.

### Example 8:

Magnetic nanoparticles solublized with polymers, lipids, detergents, small ligands, are covalently linked to organic dyes using crosslinking reagents such as EDAC, Sulfo-SMCC, and the like (see, Piercenet.com and Nature, 424 (6950): 810-816 Aug 14 2003) to form a magnetic-optical dual probe nanostructure.

Example, metallic nanoparticles (REF J. Mater. Chem., 2002, 12, 2671-2674, CHEM. COMMUN., 2002, 1334-1335, Chem. Commun., 2000, 183-184, J. AM. CHEM. SOC. 2003, 125, 14280-14281, Chem. Mater. 2004, 16, 3513-3517, J. AM. CHEM. SOC. 2004, 126, 8648-8649) solublized with polymers, lipids, detergents, small ligands, are modified on the surface using procedures discussed in Example 1-8 and to form a magnetic-optical dual probe nanostructure or a radioactive-optical dual probe nanostructure, or a tri-functional nanostructure.

### Example 9:

Magnetic nanoparticles are linked to QDs through electrostatic attraction, hydrophobic interaction, or covalent bond using crosslinking reagents such as EDAC, Sulfo-SMCC, and the like to form a magnetic-optical dual probe nanostructure (a satellite structure). In addition, magnetic particles can be attached to QD's.

### Example 10:

The multimodality nanostructures can be linked to polynucleotides, polypeptides, genes, proteins, antibodies, small-molecule drugs, PEG, dextran, and the like, for *in vivo* targeting and imaging.

### Example 11:

The magnetic-optical dual probe nanostructurecan be used as a nano-tweezer, where the magnetic property provides a handle for movements and the optical property serves as a tracer. Exemplary structures include a magnetic particle core tagged with dyes on the surface, a magnetic particle core tagged with QDs on the surface, a QD tagged with Gd³⁺ on the surface, and the like. The nano-tweezers are much smaller than the traditional optical tweezers (See, Nature, 424 (6950): 810-816 Aug 14 2003; Annu Rev Bioph Biom 23: 247-285 1994; and P Natl Acad Sci USA 94 (10): 4853-4860 May 13 1997, which are included herein by reference) and magnetic tweezers (See, Review of Scientific Instruments (2003) 74, 4158-4163; Biophysical Journal 75, 1998, 2163-2169; Review of Scientific Instruments (2003) 74, 4012-4020; and Hypertension 2001, 38, 1158-1161).

### Example 12:

Fluorescent quantum dots (QDs) are of considerable current interest for *in vivo* molecular imaging due to their unique optical properties in comparison with organic dyes, such as multicolor emission, large absorption coefficients across a wide spectral range, very high levels of brightness and photostability. However, this technology is unlikely to find broad uses in large animals limited by the intrinsic tissue penetration problem of fluorescence imaging. To address this problem, a multimodality QD probe that combines the strengths of multiple imaging modalities is described and examined. Due to the QDs' large surface area and our ability to deposit multiple other functionalities (*e.g*., magnetic contrast agents, isotopes, therapeutic drugs and *etc*.), this technology is expected to open new opportunities in ultra-sensitive and multiplexed imaging of molecular targets in living cells and animals, as well as in development of traceable delivery vehicles for therapeutics.

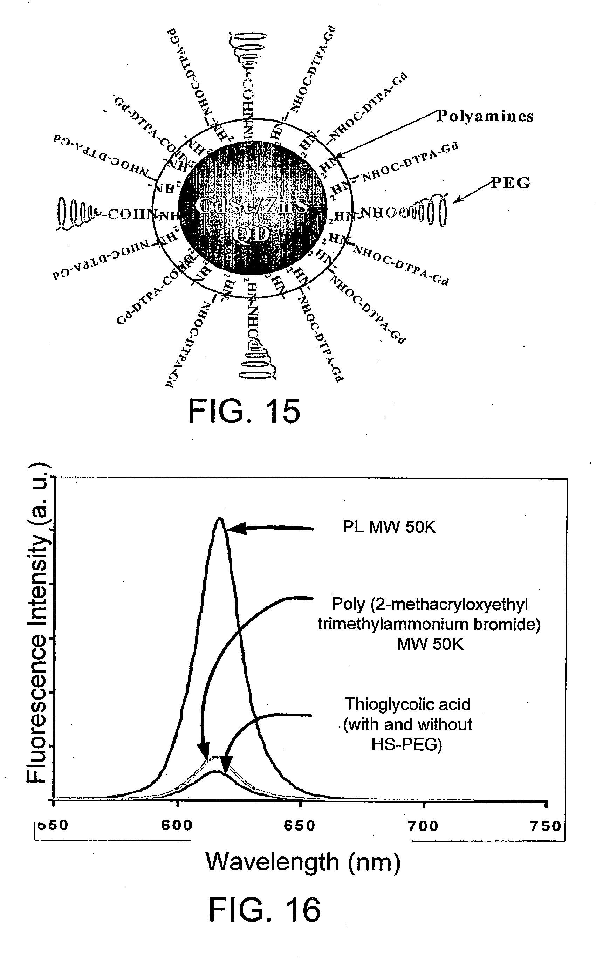

FIG. 15 shows the design of a magnetic-optical dual modality probe. This multifunctional structure was designed and prepared by using semiconductor QDs as the fluorescent probe and structural scaffold for assembly of Gd-DTPA (Diethylenetriamine pentaacetic acid-Gadolinium). To prove the concept, QDs were coated with thioglycolic acid and transferred into water following literature procedures. This ligand exchange method is easy to carry, but accompanied by a significant drop in quantum yield (typically by 90%). To restore the fluorescence and add additional imaging modalities, polylysine (PL) with an average of 340 monomer units were modified with poly(ethlene glycol) (PEG) and DTPA-Gd to a certain degree. Upon mixing with the QDs, the PEG grafted PL bind to QD surface tightly and lead to formation of highly-stable single nanoparticles (at least 100 times more stable than untreated thioglycolic acid QDs).

The polyamines enhance the fluorescence intensity of thioglycolic acid QDs by up to 10 times, as shown in FIG. 16. Although not intending to be bound by theory, the fluorescence enhancement is likely to result from the direct interaction of the primary amines with the unsaturated Cd and Zn atoms on the QD surface, and therefore reduces the possibility of charge carrier surface recombination induced by surface defects. It should also be noted, the current approach increases QD fluorescence instantly, which could lead to new development of nanoparticle-based fluorescence sensors.

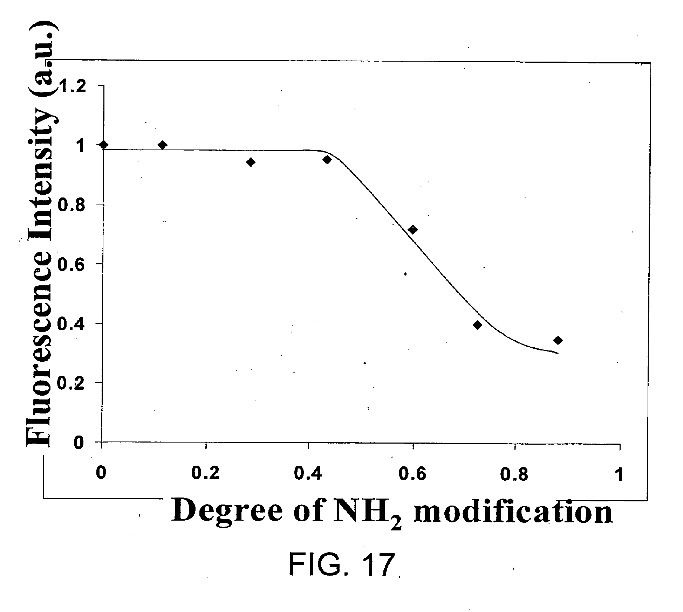

As shown in FIG. 17, PL with approximately 40% of its primary amines changed to amides still activate QD very efficiently, but higher degree of modification leads to less fluorescence enhancement. Therefore, in typical assays, 3% of amines were grafted with PEG chains for better solubility and biocompatibility, and 30% with DTPA-Gd for magnetic contrast. This elaborate design leaves 67% of the primary amines intact, which are sufficient for fluorescence enhancement. The CdSe/ZnS QDs used in this report emit at 630 nm (-5 nm diameter) were mixed with PEG-PL-Gd in a molar ratio of 1:10. After purification with ultracentrifuge, the free polymers left in the supernatant was again quantified with Ninhydrin test in comparison with the original amount of polymer added into QDs. The results show that each nanoparticle is attached with 4.87 grafted polymers on average, corresponding to 497 Gd ions. It is well documented in the literature that DTPA-Gd immobilized on macromolecules and nanoparticles provides better T1 contrast than free DTPA-Gd³⁺ of the same concentration due to faster water exchange rate. Based on this mechanism, Gd-DTPA has been linked to biocompatible polymers (such as polylysine, polysaccharide, PEG, poly(glutamic acid) and *etc*), and natural macromolecular carriers (such as albumin), for MR imaging.

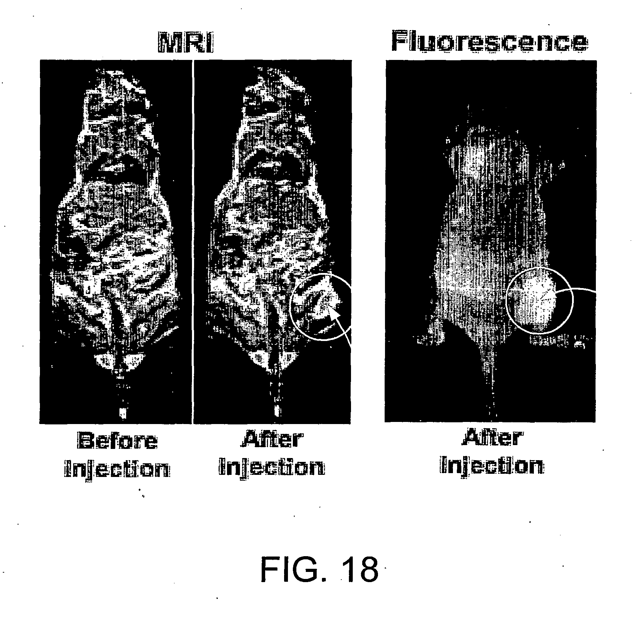

The stability of the dual-modality probe was performed. As aforementioned, the QD nanoparticle is highly water-soluble and bright due to the presence of the grafted polymer, which allows it to be dialyzed over an extended period of time without particle aggregation. After three days of dialysis, the PEG-PL-DTPA-Gd polymer coated QDs remain as a strong T1 contrast agent; however, in the control experiment (PL-PEG coated QDs mixed with free DTPA-Gd of the same concentration), only fluorescence is detectable, but not the T1 magnetic contrast. The results indicate that the probe is stable for *in vivo* molecular imaging, since most specific targeting results are accomplished within a few hours, and for PEG based long circulating probes, the images are often obtained in less than 48 hours. To demonstrate the feasibility of using QD-Gd as a dual modality probe, we injected 50 µl of 50 nM QD-Gd conjugate into mice subcutaneously. MR imaging of mice before and after injection on a clinical Philips 3T imager using standard protocols clearly marked the injection site (FIG. 18). In parallel, QD fluorescence was also detectable with a true color CCD camera and co-localized with the MR image.

In summary, the use of grafted polymer not only leads to formation of highly stable and bright QD nanoparticles, but also allows immobilization of paramagnetic compounds (such as Gd) and isotopes (*e.g*., ¹¹¹In, ⁹⁰Y, ⁹⁹Tc, ⁶⁷Ga and etc) to their surface. Due to the large surface area of nanoparticles and the versatility of chelating chemistry, tri-modality probe combining optical, magnetic and isotopic properties can be further developed. The QDs offer significant advantages over organic dyes in optical properties, whereas Gd as a T1 contrast reagent (brightening) could provide better signal/noise ratio than the SPIO T2 contrast agents (darkening effect). By linking PEG termini with targeting probes, this technology is expected to open new opportunities in ultrasensitive and multiplexed molecular imaging. For example, MRI and PET allow examination of tumors in orthotropic organs, while fluoresce is ideal for high-resolution and multicolor biopsy studies. Improvements using DTPA functionalized amphiphilic polymers and specific targeting studies are underway and will be reported in clinical journals.

It should be emphasized that the above-described embodiments of the present disclosure are merely possible examples of implementations, and are merely set forth for a clear understanding of the principles of this disclosure.

## Claims

1. A multimodality nanostructure, comprising:
at least one nanospecies having a first detectable functionality; and
a second molecule having a second detectable functionality, wherein the nanospecies is a quantum dot, and the second molecule is a contrasting agent and not a quantum dot that is attached to the nanospecies, wherein the contrasting agent is selected from the following: a magnetic compound, paramagnetic compound, radioisotope, fluorescent compound, and an organic dye, wherein the first detectable functionality and the second detectable functionality are different, and wherein the first detectable functionality is an optical functionality and the second detectable functionality is selected from the following: an optical functionality, a magnetic functionality, an x-ray functionality, and a radioactive functionality.

2. The nanostructure of claim 1, further comprising a bio-compatibility compound attached to the nanospecies, wherein the bio-compatibility is selected from the following: polyethylene glycol, polypropylene glycol 500, dextran, and polysaccharide.

3. The nanostructure of claim 2, wherein the bio-compatibility compound is a polyethylene glycol molecule having a molecular weight of 500 to 50,000 Daltons.

4. The nanostructure of claim 1, further comprising a third molecule having a third detectable functionality, wherein the third molecule is attached to the nanospecies.

5. The nanostructure of claim 4, wherein the first detectable functionality is an optical functionality, and the second detectable functionality, and the third detectable functionality are each selected from the following: an optical functionality, a magnetic functionality, an x-ray functionality, and a radioactive functionality; and wherein the first detectable functionality, the second detectable functionality, and the third detectable functionality are different.

6. The nanostructure of claim 1, wherein the second molecule is ⁶⁷Ga (gallium).

7. The nanostructure of claim 1, wherein the second molecule is selected from ¹¹¹In (indium), yttrium (⁹⁰Y), ^{99m}Tc (technetium), ²⁰¹Tl (thallium), ⁶⁷Ga (gallium), ¹²³I (iodine), ¹³¹I (iodine), ³²P (phosphorus), ¹⁵³Sm (samarium), ¹⁸⁶Re (rhenium), ¹⁸⁸Re, ¹⁶⁵Dy (dysprosium), ¹⁶⁶Ho (holmium), and combinations thereof.

8. The nanostructure of claim 1, wherein the second molecule is selected from Cr, Mn, Fe, Cu, Eu, Gd, Dy, and combinations thereof.

9. The nanostructure of claim 4, wherein the second molecule is selected from ¹¹¹In (indium), yttrium (⁹⁰Y), ^{99m}Tc (technetium), ²⁰¹Tl (thallium), ⁶⁷Ga (gallium), ¹²³I (iodine), ¹³¹I (iodine), ³²P (phosphorus), ¹⁵³Sm (samarium), ¹⁸⁶Re (rhenium), ¹⁸⁸Re, ¹⁶⁵Dy (dysprosium), ¹⁶⁶Ho (holmium), and combinations thereof; and the third molecule is selected from Cr, Mn, Fe, Cu, Eu, Gd, Dy, and combinations thereof, wherein the second molecule and third molecule are different.

10. The nanostructure of claim 1, further comprising:
a hydrophobic protection structure including at least one compound selected from a capping ligand, an amphiphilic copolymer, and combinations thereof, wherein the hydrophobic protection structure encapsulates the nanospecies; and
a bio-compatibility compound disposed substantially on the surface of the hydrophobic protection structure;
wherein the second molecule is attached to the hydrophobic protection structure,
wherein the capping ligand is selected from the following: an O=PR₃ compound, an O=PHR₂ compound, an O=PHR₁ compound, a H₂NR compound, a HNR₂ compound, a NR₃ compound, a HSR compound, and
a SR₂ compound, wherein R is a saturated or unsaturated, linear, branched, or cyclic C₁ to C₁₈ hydrocarbon residue, which is unsubstituted or substituted with halogen, and
wherein the amphiphilic copolymer is selected from the following: an amphiphilic block copolymer, an amphiphilic random copolymer, an amphiphilic alternating copolymer, and an amphiphilic periodic copolymer.

11. The nanostructure of claim 10, further comprising:
a third molecule having a third detectable functionality, wherein the third molecule is attached to the hydrophobic protection structure.

12. The nanostructure of claim 1, further comprising:
a polymer layer disposed on the surface of the nanospecies; and
a bio-compatibility compound disposed substantially on the surface of the hydrophobic protection structure;
wherein the second molecule is attached to the polymer layer.

13. The nanostructure of claim 12, further comprising:
a third molecule having a third detectable functionality, wherein the third molecule is attached to the polymer layer.

14. A method of preparing a multimodality nanostructure according to claim 1, comprising the steps of:
providing a nanospecies having a first detectable functionality; and
disposing a second molecule to the nanospecies, wherein the nanospecies is a quantum dot and the second molecule has a second detectable functionality, and wherein the first detectable functionality and the second detectable functionality are different.

15. The method of claim 14, wherein the first detectable functionality is an optical functionality and the second detectable functionality is selected from the following: an optical functionality, a magnetic functionality, an x-ray functionality, and a radioactive functionality.

16. The method of claim 14, wherein the second molecule is selected from ¹¹¹In (indium), yttrium (⁹⁰Y), ^{99m}Tc (technetium), ²⁰¹Tl (thallium), ⁶⁷Ga (gallium), ¹²³I (iodine), ¹³¹I (iodine), ³²P (phosphorus), ¹⁵³Sm (samarium), ¹⁸⁶Re (rhenium), ¹⁸⁸Re, ¹⁶⁵Dy (dysprosium), ¹⁶⁶Ho (holmium), and combinations thereof.

17. The method of claim 14, further comprising:
linking a third molecule to the nanospecies, wherein the third molecule has a third detectable functionality.

18. The method of claim 17, wherein the first detectable functionality is an optical functionality, and the second detectable functionality, and the third detectable functionality are each selected from the following: an optical functionality, a magnetic functionality, an x-ray functionality, and a radioactive functionality; and wherein the first detectable functionality, the second detectable functionality, and the third detectable functionality are different.

19. The method of claim 17, wherein the second molecule is selected from ¹¹¹In (indium), yttrium (⁹⁰Y), ^{99m}Tc (technetium), ²⁰¹Tl (thallium), ⁶⁷Ga (gallium), ¹²³I (iodine), ¹³¹I (iodine), ³²P (phosphorus), ¹⁵³Sm (samarium), ¹⁸⁶Re (rhenium), ¹⁸⁶Re, ¹⁶⁵Dy (dysprosium), ¹⁶⁶Ho (holmium), and combinations thereof; and the third molecule is selected from Cr, Mn, Fe, Cu, Eu, Gd, Dy, and combinations thereof, wherein the second molecule and third molecule are different.

20. The method of claim 14, further comprising:
linking a bio-compatibility compound to the nanospecies.

21. Use of a nanostructure according to claim 1 having:
at least one nanospecies having a first detectable functionality, a second molecule having a second detectable functionality,
wherein the nanospecies is a quantum dot and the second molecule is a contrasting agent and not a quantum dot that is attached to the nanospecies, wherein the first detectable functionality and the second detectable functionality are different, and
at least one probe attached to the nanospecies, wherein a first probe has an affinity for a target;
for the manufacture of a diagnostic agent for the detection of the target in a subject, wherein the target is selected from the following: polypeptides,
nucleic acids, polysaccharides, sugars, fatty acids, steroids, purines, pyrimidines, drugs, ligands, or combinations thereof.

22. The use of claim 21, wherein the nanostructure includes a third molecule having a third functionality, wherein the first detectable functionality, the second detectable functionality, and the third detectable functionality are different, and further comprising:
determining the presence of the target in the subject corresponding to the probe by detecting the first detectable functionality of the nanospecies, the second detectable functionality of the second molecule, and the third detectable functionality of the third molecule.

23. The use of claim 22 for the manufacture of a diagnostic agent for the detection of a cancerous disease.

24. The use of claim 21, wherein the first probe is selected from a polynucleotide, a polypeptide, an antibody, an antigen, and combinations thereof.

25. The use of claim 21, wherein the diagnostic agent is for subcutaneous injection or systemic injection.

26. The use of claim 21, wherein the nanostructure further comprises a bio-compatibility compound attached to the nanospecies, wherein the bio-compatibility is selected from the following: polyethylene glycol, polypropylene glycol 500, dextran and polysaccharide.

## Patentansprüche

1. Mutlimodalitäts-Nanostruktur umfassend:
mindestens eine Nanospezies mit einer ersten detektierbaren Funktionalität; und ein zweites Molekül mit einer zweiten detektierbaren Funktionalität, wobei die Nanospezies ein Quantenpunkt ist und das zweite Molekül ein Kontrastmittel und kein Quantenpunkt ist, welches an die Nanospezies angebracht ist, wobei das Kontrastmittel aus den Folgenden ausgewählt ist: eine magnetische Verbindung, paramagnetische Verbindung, Radioisotop, fluoreszierende Verbindung und ein organischer Farbstoff, wobei die erste detektierbare Funktionalität und die zweite detektierbare Funktionalität unterschiedlich sind und wobei die erste detektierbare Funktionalität eine optische Funktionalität ist und die zweite detektierbare Funktionalität aus den Folgenden ausgewählt ist: eine optische Funktionalität, eine magnetische Funktionalität, eine Röntgen-Funktionalität und eine radioaktive Funktionalität.

2. Nanostruktur nach Anspruch 1 weiter umfassend eine Biokompatibilitätsverbindung, welche an die Nanospezies angebracht ist, wobei die Biokompatibilitätsverbindung aus den Folgenden ausgewählt ist: Polyethylenglykol, Polypropylenglykol 500, Dextran und Polysaccharid.

3. Nanostruktur nach Anspruch 2, wobei die Biokompatibilitätsverbindung eine Polyethylenglykol-Molekül mit einem Molekulargewicht von 500 bis 50,000*Dalton ist.

4. Nanostruktur nach Anspruch 1 weiter umfassend ein drittes Molekül mit einer dritten detektierbaren Funktionalität, wobei das dritte Molekül an die Nanospezies angebracht ist.

5. Nanostruktur nach Anspruch 4, wobei die erste detektierbare Funktionalität eine optische Funktionalität ist und die zweite detektierbare Funktionalität und die dritte detektierbare Funktionalität jeweils aus den Folgenden ausgewählt sind: eine optische Funktionalität, eine magnetische Funktionalität, eine Röntgen-Funktionalität und eine radioaktive Funktionalität; und wobei die erste detektierbare Funktionalität, die zweite detektierbare Funktionalität und die dritte detektierbare Funktionalität unterschiedlich sind.

6. Nanostruktur nach Anspruch 1, wobei das zweite Molekül ⁶⁷Ga (Gallium) ist.

7. Nanostruktur nach Anspruch 1, wobei das zweite Molekül ausgewählt ist aus ¹¹¹In (Indium), Yttrium (⁹⁰Y), ^{99m}Tc (Technetium), ²⁰¹Tl (Thallium), ⁶⁷Ga (Gallium), ¹²³I (Iod), ¹³¹I (Iod), ³²P (Phosphor), ¹⁵³Sm (Samarium), ¹⁸⁶Rₑ (Rhenium), ¹⁸⁸Re, ¹⁶⁵Dy (Dysprosium), ¹⁶⁶Ho (Holmium) und Kombinationen davon.

8. Nanostruktur nach Anspruch 1, wobei das zweite Molekül ausgewählt ist aus Cr, Mn, Fe, Cu, Eu, Gd, Dy und Kombinationen davon.

9. Nanostruktur nach Anspruch 4, wobei das zweite Molekül ausgewählt ist aus ¹¹¹In (Indium), Yttrium (⁹⁰Y), ^{99m}Tc (Technetium), ²⁰¹Tl (Thallium), ⁶⁷Ga (Gallium), ¹²³I (Iod), ¹³¹I (Iod), ³²P (Phosphor), ¹⁵³Sm (Samarium), ¹⁸⁶Re (Rhenium), ¹⁸⁸Re, ¹⁶⁵Dy (Dysprosium), ¹⁶⁶Ho (Holmium) und Kombinationen davon; und das dritte Molekül ausgewählt ist aus Cr, Mn, Fe, Cu, Eu, Gd, Dy und Kombinationen davon, wobei das zweite Molekül und dritte Molekül unterschiedlich sind.

10. Nanostruktur nach Anspruch 1 weiter umfassend:
eine hydrophobe Schutzstruktur, welche mindestens eine Verbindung ausgewählt aus einem Cappingliganden, einem amphiphilen Copolymer und Kombinationen davon beinhaltet, wobei die hydrophobe Schutzstruktur die Nanospezies umhüllt; und
eine Biokompatibilitätsverbindung, welche im Wesentlichen auf der Oberfläche der hydrophoben Schutzstruktur angeordnet ist;
wobei das zweite Molekül an die hydrophobe Schutzstruktur angebracht ist,
wobei der Cappingligand aus den Folgenden ausgewählt ist: eine O=PR₃-Verbindung, eine O=PHR₂-Verbindung, eine O=PHR₁-Verbindung, eine H₂NR-Verbindung, eine HNR₂-Verbindung, eine NR₃-Verbindung, eine HSR-Verbindung und eine SR₂-Verbindung, wobei R ein gesättigter oder ungesättigter, linearer, verzweigter oder cyclischer C₁ bis C₁₈ Kohlenwasserstoffrest, welcher unsubstituiert oder mit einem Halogen substituiert ist, ist, und
wobei das amphiphile Copolymer aus den Folgenden ausgewählt ist: ein amphiphiles Blockcopolymer, ein amphiphiles zufälliges Copolymer, ein amphiphiles alternierendes Copolymer und ein amphiphiles periodisches Copolymer.

11. Nanostruktur nach Anspruch 10 weiter umfassend:
ein drittes Molekül mit einer dritten detektierbaren Funktionalität, wobei das dritte Molekül an die hydrophobe Schutzstruktur angebracht ist.

12. Nanostruktur nach Anspruch 1 weiter umfassend:
eine Polymerschicht, welche an der Oberfläche der Nanospezies angeordnet ist; und
eine Biokompatibilitätsverbindung, welche im Wesentlichen auf der Oberfläche der hydrophoben Schutzstruktur angeordnet ist;
wobei das zweite Molekül an die Polymerschicht angebracht ist.

13. Nanostruktur nach Anspruch 12 weiter umfassend:
ein drittes Molekül mit einer dritten detektierbaren Funktionalität, wobei das dritte Molekül an die Polymerschicht angebracht ist.

14. Verfahren zur Herstellung einer Multimodalitäts-Nanostruktur nach Anspruch 1 umfassend die Schritte:
Bereitstellen einer Nanospezies mit einer ersten detektierbaren Funktionalität; und Anordnen eines zweiten Moleküls auf die Nanospezies, wobei die Nanospezies ein Quantenpunkt ist und das zweite Molekül eine zweite detektierbare Funktionalität hat, und wobei die erste detektierbare Funktionalität und die zweite detektierbare Funktionalität unterschiedlich sind.

15. Verfahren nach Anspruch 14, wobei die erste detektierbare Funktionalität eine optische Funktionalität ist und die zweite detektierbare Funktionalität aus den Folgenden ausgewählt ist: eine optische Funktionalität, eine magnetische Funktionalität, eine Röntgen-Funktionalität und eine radioaktive Funktionalität.

16. Verfahren nach Anspruch 14, wobei das zweite Molekül ausgewählt ist aus ¹¹¹In (Indium), Yttrium (⁹⁰Y), ^{99m}Tc (Technetium), ²⁰¹Tl (Thallium), ⁶⁷Ga (Gallium), ¹²³I (Iod), ¹³¹I (Iod), ³²P (Phosphor), ¹⁵³Sm (Samarium), ¹⁸⁶Re (Rhenium), ¹⁸⁸Re, ¹⁶⁵Dy (Dysprosium), ¹¹⁶Ho (Holmium) und Kombinationen davon.

17. Verfahren nach Anspruch 14 weiter umfassend:
Verknüpfen eines dritten Moleküls an die Nanospezies, wobei das dritte Molekül eine dritte detektierbare Funktionalität hat.

18. Verfahren nach Anspruch 17, wobei die erste detektierbare Funktiönalität eine optische Funktionalität ist und die zweite detektierbare Funktionalität und die dritte detektierbare Funktionalität jeweils aus den Folgenden ausgewählt sind: eine optische Funktionalität, eine magnetische Funktionalität, eine Röntgen-Funktionalität und eine radioaktive Funktionalität; und wobei die erste detektierbare Funktionalität, die zweite detektierbare Funktionalität und die dritte detektierbare Funktionalität unterschiedlich sind.

19. Verfahren nach Anspruch 17, wobei das zweite Molekül ausgewählt ist aus ¹¹¹In (Indium), Yttrium (⁹⁰Y), ^{99m}Tc (Technetium), ²⁰¹Tl (Thallium), ⁶⁷Ga (Gallium), ¹²³I (Iod), ¹³¹I (Iod), ³²P (Phosphor), ¹⁵³Sm (Samarium), ¹⁸⁶Re (Rhenium), ¹⁸⁸Re, ¹⁶⁵Dy (Dysprosium), ¹⁶⁶Ho (Holmium) und Kombinationen davon; und das dritte Molekül ausgewählt ist aus Cr, Mn, Fe, Cu, Eu, Gd, Dy und Kombinationen davon, wobei das zweite Molekül und dritte Molekül unterschiedlich sind.

20. Verfahren nach Anspruch 14 weiter umfassend:
Verknüpfen einer Biokompatibilitätsverbindung an die Nanospezies.

21. Verwendung einer Nanostruktur nach Anspruch 1 mit:
mindestens einer Nanospezies mit einer ersten detektierbaren Funktionalität, einem zweiten Molekül mit einer zweiten detektierbaren Funktionalität,
wobei die Nanospezies ein Quantenpunkt ist und das zweite Molekül ein Kontrastmittel und kein Quantenpunkt ist, welches an die Nanospezies angebracht ist, wobei die erste detektierbare Funktionalität und die zweite detektierbare Funktionalität unterschiedlich sind, und
mindestens eine Sonde, welche an die Nanospezies angebracht ist, wobei eine erste Sonde eine Affinität für ein Target hat;
zur Herstellung eines diagnostischen Wirkstoffes für die Detektion des Ziels in einem Individuum, wobei das Ziel aus den Folgenden ausgewählt ist: Polypeptide, Nucleinsäuren, Polysaccharide, Zucker, Fettsäuren, Steroide, Purine, Pyrimidine, Medikamente, Liganden oder Kombinationen davon.

22. Verwendung nach Anspruch 21, wobei die Nanostruktur ein drittes Molekül mit einer dritten Funktionalität beinhaltet, wobei die erste detektierbare Funktionalität, die zweite detektierbare Funktionalität und die dritte detektierbare Funktionalität unterschiedlich sind, und weiter umfassend:
Bestimmen des Vorhandenseins des Targets in dem Individuum entsprechend der Sonde mittels Detektieren der ersten detektierbaren Funktionalität der Nanospezies, der zweiten detektierbaren Funktionalität des zweiten Moleküls und der dritten detektierbaren Funktionalität des dritten Moleküls.

23. Verwendung nach Anspruch 22 für die Herstellung eines diagnostischen Wirkstoffes für die Detektion einer Krebserkrankung.

24. Verwendung nach Anspruch 21, wobei die erste Sonde ausgewählt ist aus einem Polynukleotid, einem Polypeptid, einem Antikörper, einem Antigen und Kombinationen davon.

25. Verwendung nach Anspruch 21, wobei der diagnostische Wirkstoff für subkutane Injektion oder systemische Injektion ist.

26. Verwendung nach Anspruch 21, wobei die Nanostruktur weiter eine Biokompatibilitätsverbindung, welche an die Nanospezies angebracht ist, umfasst, wobei die Biokompatibilitätsverbindung aus den Folgenden ausgewählt ist: Polytehylenglykol, Polypropylenglykol 500, Dextran und Polysaccharid.

## Revendications

1. Nanostructure multimodale, comprenant :
au moins une nano-espèce ayant une première fonctionnalité détectable, et
une deuxième molécule ayant une deuxième fonctionnalité détectable, où la nano-espèce est un point quantique, et la deuxième molécule est un agent de contraste et pas un point quantique qui est attaché à la nano-espèce, où l'agent de contraste est choisi parmi les suivants : un composé magnétique, un composé paramagnétique, un radio-isotope, un composé fluorescent, et un colorant organique, où la première fonctionnalité détectable et la deuxième fonctionnalité détectable sont différentes, et où la première fonctionnalité détectable est une fonctionnalité optique et la deuxième fonctionnalité détectable est choisie parmi les suivantes : une fonctionnalité optique, une fonctionnalité magnétique, une fonctionnalité rayons X et une fonctionnalité radioactive.

2. Nanostructure selon la revendication 1, comprenant en outre un composé de biocompatibilité attaché à la nano-espèce, où le composé de biocompatibilité est choisi parmi les suivantes : polyéthylèneglycol, polypropylèneglycol 500, dextrane et polysaccharide.

3. Nanostructure selon la revendication 2, où le composé de biocompatibilité est une molécule de polyéthylèneglycol ayant un poids moléculaire allant de 500 à 50 000 Daltons.

4. Nanostructure selon la revendication 1, comprenant en outre une troisième molécule ayant une troisième fonctionnalité détectable, où la troisième molécule est attachée à la nano-espèce.

5. Nanostructure selon la revendication 4, où la première fonctionnalité détectable est une fonctionnalité optique, et la deuxième fonctionnalité détectable et la troisième fonctionnalité détectable sont chacune choisies parmi les suivantes : une fonctionnalité optique, une fonctionnalité magnétique, une fonctionnalité rayons X et une fonctionnalité radioactive, et où la première fonctionnalité détectable, la deuxième fonctionnalité détectable et la troisième fonctionnalité détectable sont différentes.

6. Nanostructure selon la revendication 1, où la deuxième molécule est ⁶⁷Ga (gallium).

7. Nanostructure selon la revendication 1, où la deuxième molécule est choisie parmi ¹¹¹In (indium), yttrium (⁹⁰Y), ^{99m}Tc (technétium), ²⁰¹Tl (thallium), ⁶⁷Ga (gallium), ¹²³I (iode), ¹³¹I (iode), ³²P (phosphore), ¹⁵³Sm (samarium), ¹⁸⁶Re (rhénium), ¹⁸⁸Re, ¹⁶⁵Dy (dysprosium), ¹⁶⁶Ho (holmium) et leurs combinaisons.

8. Nanostructure selon la revendication 1, où la deuxième molécule est choisie parmi Cr, Mn, Fe, Cu, Eu, Gd, Dy et leurs combinaisons.

9. Nanostructure selon la revendication 4, où la deuxième molécule est choisie parmi ¹¹¹In (indium), yttrium (⁹⁰Y), ^{99m}Tc (technétium), ²⁰¹Tl (thallium), ⁶⁷Ga (gallium), ¹²³I (iode), ¹³¹I (iode), ³²P (phosphore), ¹⁵³Sm (samarium), ¹⁸⁶Re (rhénium), ¹⁸⁸Re, ¹⁶⁵Dy (dysprosium), ¹⁶⁶Ho (holmium) et leurs combinaisons, et la troisième molécule est choisie parmi Cr, Mn, Fe, Cu, Eu, Gd, Dy et leurs combinaisons, où la deuxième molécule et la troisième molécule sont différentes.

10. Nanostructure selon la revendication 1, comprenant en outre :
une structure hydrophobe de protection comprenant au moins un composé choisi parmi un ligand de coiffage, un copolymère amphiphile et leurs combinaisons, où la structure hydrophobe de protection encapsule la nano-espèce, et
un composé de biocompatibilité disposé sensiblement sur la surface de la structure hydrophobe de protection,
où la deuxième molécule est attachée à la structure hydrophobe de protection,
où le ligand de coiffage est choisi parmi les suivants : un composé O=PR₃, un composé O=PHR₂, un composé O=PHR₁, un composé H₂NR, un composé HNR₂, un composé NR₃, un composé HSR et un composé SR₂, où R est un résidu hydrocarboné en C₁ à C₁₈ saturé ou insaturé, linéaire, ramifié ou cyclique, qui est non substitué ou substitué par halogène, et
où le copolymère amphiphile est choisi parmi les suivants : un copolymère séquence amphiphile, un copolymère statistique amphiphile, un copolymère alternant amphiphile et un copolymère périodique amphiphile.

11. Nanostructure selon la revendication 10, comprenant en outre :
une troisième molécule ayant une troisième fonctionnalité détectable, où la troisième molécule est attachée à la structure hydrophobe de protection.

12. Nanostructure selon la revendication 1, comprenant en outre :
une couche polymère disposée sur la surface de la nano-espèce, et
un composé de biocompatibilité disposé sensiblement sur la surface de la structure hydrophobe de protection,
où la deuxième molécule est attachée à la couche polymère.

13. Nanostructure selon la revendication 12, comprenant en outre :
une troisième molécule ayant une troisième fonctionnalité détectable, où la troisième molécule est attachée à la couche polymère.

14. Procédé de préparation d'une nanostructure multimodale selon la revendication 1, comprenant les étapes consistant à :
disposer une nano-espèce ayant une première fonctionnalité détectable, et
disposer une deuxième molécule sur la nano-espèce, où la nano-espèce est un point quantique et la deuxième molécule a une deuxième fonctionnalité détectable, et où la première fonctionnalité détectable et la deuxième fonctionnalité détectable sont différentes.

15. Procédé selon la revendication 14, où la première fonctionnalité détectable est une fonctionnalité optique et la deuxième fonctionnalité détectable est choisie parmi les suivantes : une fonctionnalité optique, une fonctionnalité magnétique, une fonctionnalité rayons X et une fonctionnalité radioactive.

16. Procédé selon la revendication 14, où la deuxième molécule est choisie parmi ¹¹¹In (indium), yttrium (⁹⁰Y), ^{99m}Tc (technétium), ²⁰¹Tl (thallium), ⁶⁷Ga (gallium), ¹²³I (iode), ¹³¹I (iode), ³²P (phosphore), ¹⁵³Sm (samarium), ¹⁸⁶Re (rhénium), ¹⁸⁸Re, ¹⁶⁵Dy (dysprosium), ¹⁶⁶Ho (holmium) et leurs combinaisons.

17. Procédé selon la revendication 14, comprenant en outre :
la liaison d'une troisième molécule à la nano-espèce, où la troisième molécule a une troisième fonctionnalité détectable.

18. Procédé selon la revendication 17, où la première fonctionnalité détectable est une fonctionnalité optique, et la deuxième fonctionnalité détectable et la troisième fonctionnalité détectable sont chacune choisies parmi les suivantes : une fonctionnalité optique, une fonctionnalité magnétique, une fonctionnalité rayons X et une fonctionnalité radioactive, et où la première fonctionnalité détectable, la deuxième fonctionnalité détectable et la troisième fonctionnalité détectable sont différentes.

19. Procédé selon la revendication 17, où la deuxième molécule est choisie parmi ¹¹¹In (indium), yttrium (⁹⁰Y), ^{99m}Tc (technétium), ²⁰¹Tl (thallium), ⁶⁷Ga (gallium), ¹²³I (iode), ¹³¹I (iode), ³²P (phosphore), ¹⁵³Sm (samarium), ¹⁸⁶Re (rhénium), ¹⁸⁸Re, ¹⁶⁵Dy (dysprosium), ¹⁶⁶Ho (holmium) et leurs combinaisons, et la troisième molécule est choisie parmi Cr, Mn, Fe, Cu, Eu, Gd, Dy et leurs combinaisons, où la deuxième molécule et la troisième molécule sont différentes.

20. Procédé selon la revendication 14, comprenant en outre :
la liaison d'un composé de biocompatibilité à la nano-espèce.

21. Utilisation d'une nanostructure selon la revendication 1, ayant :
au moins une nano-espèce ayant une première fonctionnalité détectable, une deuxième molécule ayant une deuxième fonctionnalité détectable,
où la nano-espèce est un point quantique, et la deuxième molécule est un agent de contraste et pas un point quantique qui est attaché à la nano-espèce, où la première fonctionnalité détectable et la deuxième fonctionnalité détectable sont différentes, et
au moins une sonde attachée à la nano-espèce, où une première sonde a une affinité pour une cible ;
pour la préparation d'un agent diagnostique pour la détection de la cible chez un sujet, où la cible est choisie parmi les suivantes : polypeptides, acides nucléiques, polysaccharides, sucres, acides gras, stéroïdes, purines, pyrimidines, médicaments, ligands ou combinaisons de ceux-ci.

22. Utilisation selon la revendication 21, où la nanostructure comprend une troisième molécule ayant une troisième fonctionnalité, où la première fonctionnalité détectable, la deuxième fonctionnalité détectable et la troisième fonctionnalité détectable sont différentes, et comprenant en outre :
la détermination de la présence de la cible chez le sujet correspondant à la sonde par détection de la première fonctionnalité détectable de la nano-espèce, la deuxième fonctionnalité détectable de la deuxième molécule, et la troisième fonctionnalité détectable de la troisième molécule.

23. Utilisation selon la revendication 22, pour la préparation d'un agent diagnostique pour la détection d'une maladie cancéreuse.

24. Utilisation selon la revendication 21, où la première sonde est choisie parmi un polynucléotide, un polypeptide, un anticorps, un antigène, et leurs combinaisons.

25. Utilisation selon la revendication 21, où l'agent diagnostique est destiné à une injection sous-cutanée ou une injection systémique.

26. Utilisation selon la revendication 21, où la nanostructure comprend en outre, un composé de biocompatibilité attaché à la nano-espèce, où le composé de biocompatibilité est choisi parmi les suivants : polyéthylèneglycol, polypropylèneglycol 500, dextrane et polysaccharide.
